# EUROPEAN PATENT APPLICATION

(11) **EP 4 745 231 A1**
(43) Date of publication of application: **20.05.2026**
(21) Application number: 24843086.0
(22) Date of filing: 12.07.2024
(51) Int. Cl.: C12N 7/01, A61K 35/76, A61K 38/43, A61K 47/62, A61K 47/65, A61K 48/00, A61P 11/00, A61P 31/04, A61P 43/00

(54) **MODIFIED BACTERIOPHAGE**

(30) Priority: 14.07.2023 JP 2023116013
(71) Applicant: Arrowsmith Inc., Tsukuba-shi, Ibaraki 305-0841 (JP)
(72) Inventor: MATSUOKA, Hideaki, Tokyo 103-8411 (JP); MATSUI, Toshiyuki, Ibaraki 300-0845 (JP); SAKAI, Seishin, Tokyo 103-8411 (JP)
(74) Representative: Hoffmann Eitle
(86) International application number: PCT/JP2024/025265
(87) International publication number: WO 2025/018295

(57) **Abstract**

The present invention provides a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513 or NITE BP-03514, or Accession No. NITE BP-03918, and the capsid of the bacteriophage is linked to a cell-penetrating peptide via a tag.

## Description

### Technical Field

The present invention relates to a new bacteriophage and a modified bacteriophage, and uses of these bacteriophages.

### Background Art

Non-tuberculous mycobacteria (NTM) disease is an infection by acid-fast bacteria other than *Mycobacterium tuberculosis* and *Mycobacterium leprae.* NTM is environment resident bacteria that exist in lakes, marshes, soil, and the like, and causes respiratory symptoms when inhaled as an aerosol or the like. Examples of NTM *include Mycobacterium avium* (*M. avium*), *Mycobacterium intracellulare (M. intracellulare*), *Mycobacterium fortuitum* (*M. fortuitum*), *Mycobacterium chelonae* (*M. chelonae*), *Mycobacterium gordonae* (*M. gordonae*), *Mycobacterium szulagai* (*M. szulgai*), *Mycobacterium kansasii* (*M. kansasii*), and *Mycobacterium genavense* (*M. genavense*) (Infection. 2004; 32: 257.). Among them, infections caused by *Mycobacterium avium* complex (MAC) including *Mycobacterium avium* and *Mycobacterium intracellulare* are called MAC disease, of which pulmonary infections are particularly called pulmonary MAC disease.

Therapeutic methods for bacterial infections including NTM disease are generally multidrug therapy containing macrolide antibiotics, but in recent years, phage therapy using the lytic activity of a bacteriophage (hereinafter also referred to as a phage) has been known (Microorganisms. 2021; 9 (3): 596., WO2021/092362 A). For example, D29, TM4, and ZoeJ have been reported as phages having lytic activity against *Mycobacterium avium* (Journal of Medical Microbiology 2006;55(1):37.; Microorganisms. 2021; 9 (3): 596.; Microb. Drug Resist. 2006; 12: 1.).

A bacteriophage is a virus whose host is a bacterium. A phage that infects a specific bacterium injects its own genome into the bacterium and uses the metabolic mechanism of the host bacterium to propagate its progeny phage. Thereafter, the bacterial cell wall is destructed by the lytic enzymes carried in the phage genome, the progeny phages are released, and the host bacterium is killed.

Phages can be broadly classified into non-temperate phages (lytic phages) and temperate phages (lysogenic phages) (Scientifica (Cairo). 2014; 2014: 581639.). Non-temperate phages replicate themselves without integrating into the host bacterial DNA. Progeny phages are propagated within the bacterium, resulting in lysis of the bacterium and release of mature phage particles. Meanwhile, after infection to bacteria, temperate phages integrate into the host bacterial DNA and replicate themselves together with the host bacterial DNA. It is harmless to the host bacterium just by integrating into the host bacterial DNA, and it is possible to maintain a lysogenized state (a state in which the phage DNA integrates into the host bacterial DNA and replicates itself with the host bacterial DNA). However, when temperate phages are exposed to an external stimulus such as ultraviolet rays, the temperate phages release a lytic enzyme like non-temperate phages, lyse the host bacterium, and release mature phage particles.

Phages lacking genes involved in elements of lysogenization have also been reported. For example, a phage lacking integrase gene (Nat Med. 2019; 25 (5): 730.) and a phage lacking integrase gene and immunity repressor gene (mBio. 2021; 12 (3): e00973.) have been reported.

A phage is composed of a head (a capsid that stores the phage genome) and a tail. The phage capsid is produced by synthesizing, in a host bacterium, a plurality of types of proteins constituting the capsid from the phage genome, and then assembling these capsid-constituting proteins. A phage in which a peptide or protein of interest is added to the capsid thereof is produced by introducing, into a host bacterium, a plasmid that has a DNA sequence encoding the peptide or protein of interest ligated to a DNA sequence encoding a given capsid structural protein, along with the phage genome. For example, it has been reported that a modified TM4 phage having a capsid protein fused to an S-tag peptide can be obtained by infecting, with a wild-type TM4 phage, a genetically modified host bacterium, which harbors a plasmid where a DNA sequence encoding the S-tag peptide is ligated downstream of a DNA sequence encoding the capsid structural protein of the wild-type TM4 phage (Non Patent Literature 1).

It has been known that MAC can intracellularly infect. If a phage can be efficiently delivered into cells, the phage will be able to lyse MAC within the cells, and is therefore expected to possess a stronger therapeutic effect. As one effective means for delivering bacteriophages into cells, use of a cell-penetrating peptide (CPP) is being discussed (Non Patent Literature 2).

Numerous sequences of CPPs are known. For example, it has been reported that Inv3, a peptide contained within Mycobacterium cell entry protein (Mce1A) that is a protein derived from *Mycobacterium tuberculosis,* possesses CPP activity (Non Patent Literature 3).

### Citation List

### Non Patent Literature

Non Patent Literature 1: Appl Environ Microbiol. 2013; 79 (18): 5608
Non Patent Literature 2: Adv Drug Deliv Rev. 2021; 176: 113864
Non Patent Literature 3: Anal. Biochem. 2006; 353 (1): 7

### Summary of Invention

### Technical Problem

An object of the present invention is to provide a bacteriophage having lytic activity against *Mycobacterium avium* and/or *Mycobacterium intracellulare.* It is another object of the present invention to provide a means and a method, particularly a bacteriophage and a pharmaceutical composition, for preventing or treating nontuberculous mycobacterial disease such as pulmonary MAC disease.

### Solution to Problem

As a result of creative studies in producing a bacteriophage, the present inventors have obtained a new bacteriophage (Example 1), and have found that the bacteriophage possesses resistance to freeze-thaw (Example 2). Besides, the inventors have obtained a bacteriophage having a modified capsid (Example 3). Furthermore, it has been found that the new bacteriophage and the modified bacteriophage possess lytic activity against *Mycobacterium avium* and *Mycobacterium intracellulare* (Examples 4 to 5). In addition, the present inventors have found that a pharmaceutical composition containing a combination of specific bacteriophage strains possesses excellent bactericidal activity (Example 6). Based on these findings, the present invention has been accomplished.

Specifically, the present invention may include the following inventions as materials or methods that are medically or industrially useful.
[1] A bacteriophage having lytic activity against *Mycobacterium avium* and/or *Mycobacterium intracellulare,*
   wherein a genome of the bacteriophage: (a) comprises a nucleic acid sequence having 90% or more identity to a nucleic acid sequence of a genome of a bacteriophage identified by Accession No. NITE BP-03513, and (b) lacks an integrase gene and/or an immunity repressor gene, and
   a capsid of the bacteriophage is linked to an S-tag.
[2] The bacteriophage according to [1], wherein the genome of the bacteriophage comprises the nucleic acid sequence of the genome of the bacteriophage identified by Accession No. NITE BP-03513.
[3] The bacteriophage according to [1] or [2], wherein the genome of the bacteriophage consists of the nucleic acid sequence of the genome of the bacteriophage identified by Accession No. NITE BP-03513.
[4] A bacteriophage having lytic activity against *Mycobacterium avium* and/or *Mycobacterium intracellulare,* wherein the bacteriophage is a bacteriophage identified by Accession No. NITE BP-03513 or a passage strain thereof, and a capsid of the bacteriophage or the passage strain thereof is linked to an S-tag.
[5] The bacteriophage according to any one of [1] to [4], wherein the capsid is linked further to a cell-penetrating peptide via the S-tag.
[6] The bacteriophage according to [5], wherein the cell-penetrating peptide includes Inv3.
[7] A pharmaceutical composition comprising the bacteriophage according to any one of [1] to [6], and a pharmaceutically acceptable excipient.
[8] The pharmaceutical composition according to [7], further comprising one or more strains of a bacteriophage having lytic activity against *Mycobacterium avium* and/or *Mycobacterium intracellulare.*
[9] A bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03918, and (b) lacks an integrase gene and/or an immunity repressor gene.
[10] The bacteriophage according to [9], which possesses resistance to freeze-thaw.
[11] The bacteriophage according to [9], wherein the genome of the bacteriophage comprises the nucleic acid sequence of the genome of the bacteriophage identified by Accession No. NITE BP-03918.
[12] The bacteriophage according to [9] or [11], wherein the genome of the bacteriophage consists of the nucleic acid sequence of the genome of the bacteriophage identified by Accession No. NITE BP-03918.
[13] A bacteriophage having lytic activity against *Mycobacterium avium* and/or*Mycobacterium intracellulare,* wherein the bacteriophage is a bacteriophage identified by Accession No. NITE BP-03918 or a passage strain thereof.
[14] The bacteriophage according to any one of [9] to [13], wherein a capsid of the bacteriophage is linked to a tag.
[15] The bacteriophage according to any one of [9] to [13], wherein a capsid of the bacteriophage is linked to a cell-penetrating peptide via a tag.
[16] The bacteriophage according to [14] or [15], wherein the tag is an S-tag.
[17] The bacteriophage according to [15] or [16], wherein the cell-penetrating peptide includes Inv3.
[18] A pharmaceutical composition comprising the bacteriophage according to any one of [9] to [17], and a pharmaceutically acceptable excipient.
[19] The pharmaceutical composition according to [18], further comprising one or more strains of a bacteriophage having lytic activity against *Mycobacterium avium* and/or *Mycobacterium intracellulare.*
[20] The pharmaceutical composition according to [7], comprising the bacteriophage according to any one of [1] to [6], and further comprising one or more bacteriophages selected from the group consisting of bacteriophages of the following (1) to (3):
   (1) (A) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03514, and (b) lacks an integrase gene and/or an immunity repressor gene, or (B) the bacteriophage (A) in which a capsid of the bacteriophage is linked to a cell-penetrating peptide via a tag;
   (2) (C) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03918, and (b) lacks an integrase gene and/or an immunity repressor gene, or (D) the bacteriophage (C) in which a capsid of the bacteriophage is linked to a cell-penetrating peptide via a tag; and
   (3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of a bacteriophage identified by Accession No. NITE BP-03519.
[21] The pharmaceutical composition according to [7], comprising the bacteriophage according to any one of [1] to [6], and further comprising one or more bacteriophages selected from the group consisting of bacteriophages of the following (1) to (3):
   (1) (A) a bacteriophage having lytic activity against *Mycobacterium avium* and/or *Mycobacterium intracellulare,* wherein a genome of the bacteriophage comprises a nucleic acid sequence of a genome of a bacteriophage identified by Accession No. NITE BP-03514, or (B) the bacteriophage (A) in which a capsid of the bacteriophage is linked to a cell-penetrating peptide via a tag;
   (2) (C) a bacteriophage having lytic activity against *Mycobacterium avium* and/or *Mycobacterium intracellulare,* wherein a genome of the bacteriophage comprises a nucleic acid sequence of a genome of a bacteriophage identified by Accession No. NITE BP-03918, or (D) the bacteriophage (C) in which a capsid of the bacteriophage is linked to a cell-penetrating peptide via a tag; and
   (3) a bacteriophage having lytic activity against *Mycobacterium avium* and/or *Mycobacterium intracellulare,* wherein a genome of the bacteriophage comprises a nucleic acid sequence of a genome of a bacteriophage identified by Accession No. NITE BP-03519.
[22] The pharmaceutical composition according to [7], comprising the bacteriophage according to any one of [1] to [6], and further comprising one or more bacteriophages selected from the group consisting of bacteriophages of the following (1) to (3):
   (1) (A) a bacteriophage having lytic activity against *Mycobacterium avium* and/or *Mycobacterium intracellulare,* wherein a genome of the bacteriophage consists of a nucleic acid sequence of a genome of a bacteriophage identified by Accession No. NITE BP-03514, or (B) the bacteriophage (A) in which a capsid of the bacteriophage is linked to a cell-penetrating peptide via a tag;
   (2) (C) a bacteriophage having lytic activity against *Mycobacterium avium* and/or *Mycobacterium intracellulare,* wherein a genome of the bacteriophage consists of a nucleic acid sequence of a genome of a bacteriophage identified by Accession No. NITE BP-03918, or (D) the bacteriophage (C) in which a capsid of the bacteriophage is linked to a cell-penetrating peptide via a tag; and
   (3) a bacteriophage having lytic activity against *Mycobacterium avium* and/or *Mycobacterium intracellulare,* wherein a genome of the bacteriophage consists of a nucleic acid sequence of a genome of a bacteriophage identified by Accession No. NITE BP-03519.
[23] The pharmaceutical composition according to [7], comprising the bacteriophage according to any one of [1] to [6], and further comprising one or more bacteriophages selected from the group consisting of bacteriophages of the following (1) to (3):
   (1) (A) a bacteriophage having lytic activity against *Mycobacterium avium* and/or *Mycobacterium intracellulare,* wherein the bacteriophage is a bacteriophage identified by Accession No. NITE BP-03514 or a passage strain thereof, or (B) the bacteriophage (A) in which a capsid of the bacteriophage or the passage strain thereof is linked to a cell-penetrating peptide via a tag;
   (2) (C) a bacteriophage having lytic activity against *Mycobacterium avium* and/or *Mycobacterium intracellulare,* wherein the bacteriophage is a bacteriophage identified by Accession No. NITE BP-03918 or a passage strain thereof, or (D) the bacteriophage (C) in which a capsid of the bacteriophage or the passage strain thereof is linked to a cell-penetrating peptide via a tag; and
   (3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.
[24] A pharmaceutical composition comprising bacteriophages of the following (1) to (3), and a pharmaceutically acceptable excipient:
   (1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03513, and (b) lacks an integrase gene and/or an immunity repressor gene, and a capsid of the bacteriophage is linked to Inv3 via an S-tag;
   (2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03514, and (b) lacks an integrase gene and/or an immunity repressor gene; and
   (3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03918, and (b) lacks an integrase gene and/or an immunity repressor gene, and a capsid of the bacteriophage is linked to Inv3 via an S-tag.
[25] The pharmaceutical composition according to [24], comprising bacteriophages of the following (1) to (3):
   (1) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03513, and the capsid of the bacteriophage is linked to Inv3 via an S-tag;
   (2) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03514; and
   (3) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03918, and the capsid of the bacteriophage is linked to Inv3 via an S-tag.
[26] The pharmaceutical composition according to [24] or [25], comprising bacteriophages of the following (1) to (3):
   (1) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03513, and the capsid of the bacteriophage is linked to Inv3 via an S-tag;
   (2) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03514; and
   (3) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03918, and the capsid of the bacteriophage is linked to Inv3 via an S-tag.
[27] A pharmaceutical composition comprising bacteriophages of the following (1) to (3), and a pharmaceutically acceptable excipient:
   (1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and a capsid of the bacteriophage or the passage strain thereof is linked to Inv3 via an S-tag;
   (2) a bacteriophage having lytic activity against *Mycobacterium avium* and/or *Mycobacterium intracellulare,* wherein the bacteriophage is a bacteriophage identified by Accession No. NITE BP-03514 or a passage strain thereof; and
   (3) a bacteriophage having lytic activity against *Mycobacterium avium* and/or *Mycobacterium intracellulare,* wherein the bacteriophage is a bacteriophage identified by Accession No. NITE BP-03918 or a passage strain thereof, and a capsid of the bacteriophage or the passage strain thereof is linked to Inv3 via an S-tag.
[28] A pharmaceutical composition comprising bacteriophages of the following (1) to (3), and a pharmaceutically acceptable excipient:
   (1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03513, and (b) lacks an integrase gene and/or an immunity repressor gene, and a capsid of the bacteriophage is linked to Inv3 via an S-tag;
   (2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03918, and (b) lacks an integrase gene and/or an immunity repressor gene, and a capsid of the bacteriophage is linked to Inv3 via an S-tag; and
   (3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of a bacteriophage identified by Accession No. NITE BP-03519.
[29] The pharmaceutical composition according to [28], comprising bacteriophages of the following (1) to (3):
   (1) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03513, and the capsid of the bacteriophage is linked to Inv3 via an S-tag;
   (2) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03918, and the capsid of the bacteriophage is linked to Inv3 via an S-tag; and
   (3) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03519.
[30] The pharmaceutical composition according to [28] or [29], comprising bacteriophages of the following (1) to (3):
   (1) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03513, and the capsid of the bacteriophage is linked to Inv3 via an S-tag;
   (2) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03918, and the capsid of the bacteriophage is linked to Inv3 via an S-tag; and
   (3) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03519.
[31] A pharmaceutical composition comprising bacteriophages of the following (1) to (3), and a pharmaceutically acceptable excipient:
   (1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and a capsid of the bacteriophage or the passage strain thereof is linked to Inv3 via an S-tag;
   (2) a bacteriophage having lytic activity against *Mycobacterium avium* and/or *Mycobacterium intracellulare,* wherein the bacteriophage is a bacteriophage identified by Accession No. NITE BP-03918 or a passage strain thereof, and a capsid of the bacteriophage or the passage strain thereof is linked to Inv3 via an S-tag; and
   (3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.
[32] A pharmaceutical composition comprising bacteriophages of the following (1) to (3), and a pharmaceutically acceptable excipient:
   (1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03513, and (b) lacks an integrase gene and/or an immunity repressor gene, and a capsid of the bacteriophage is linked to Inv3 via an S-tag;
   (2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03514, and (b) lacks an integrase gene and/or an immunity repressor gene; and
   (3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of a bacteriophage identified by Accession No. NITE BP-03519.
[33] The pharmaceutical composition according to [32], comprising bacteriophages of the following (1) to (3):
   (1) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03513, and the capsid of the bacteriophage is linked to Inv3 via an S-tag;
   (2) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03514; and
   (3) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03519.
[34] The pharmaceutical composition according to [32] or [33], comprising bacteriophages of the following (1) to (3):
   (1) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03513, and the capsid of the bacteriophage is linked to Inv3 via an S-tag;
   (2) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03514; and
   (3) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03519.
[35] A pharmaceutical composition comprising bacteriophages of the following (1) to (3), and a pharmaceutically acceptable excipient:
   (1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and a capsid of the bacteriophage or the passage strain thereof is linked to Inv3 via an S-tag;
   (2) a bacteriophage having lytic activity against *Mycobacterium avium* and/or *Mycobacterium intracellulare,* wherein the bacteriophage is a bacteriophage identified by Accession No. NITE BP-03514 or a passage strain thereof; and
   (3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.
[36] The pharmaceutical composition according to any one of [7], [8], [18], [19], and [20] to [35], which is a pharmaceutical composition for preventing or treating nontuberculous mycobacterial disease.
[37] The pharmaceutical composition according to [36], wherein the nontuberculous mycobacterial disease is pulmonary MAC disease.

### Advantageous Effects of Invention

The bacteriophage, the modified bacteriophage, and the pharmaceutical composition of the present invention exhibit lytic activity against *Mycobacterium avium* and/or*Mycobacterium intracellulare,* and the bacteriophage, the modified bacteriophage, and the pharmaceutical composition of the present invention are expected to be useful for preventing or treating nontuberculous mycobacterial diseases such as pulmonary MAC disease.

### Brief Description of Drawings

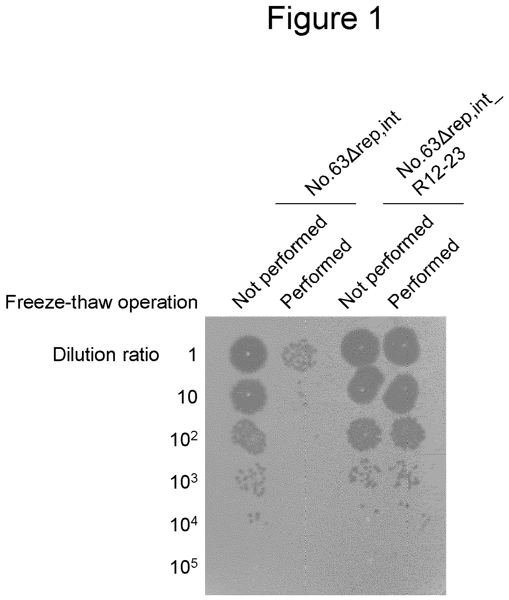

[Figure 1] Figure 1 shows a photograph showing the results of a plaque formation assay for comparing the influence, on the number of phages, of physical damage caused by freeze-thaw operations, for #63 lysogenic gene-deficient strain (No. 63Δrep, int) and #63 freeze-thaw resistant strain (No. 63Δrep, int_R12-23).

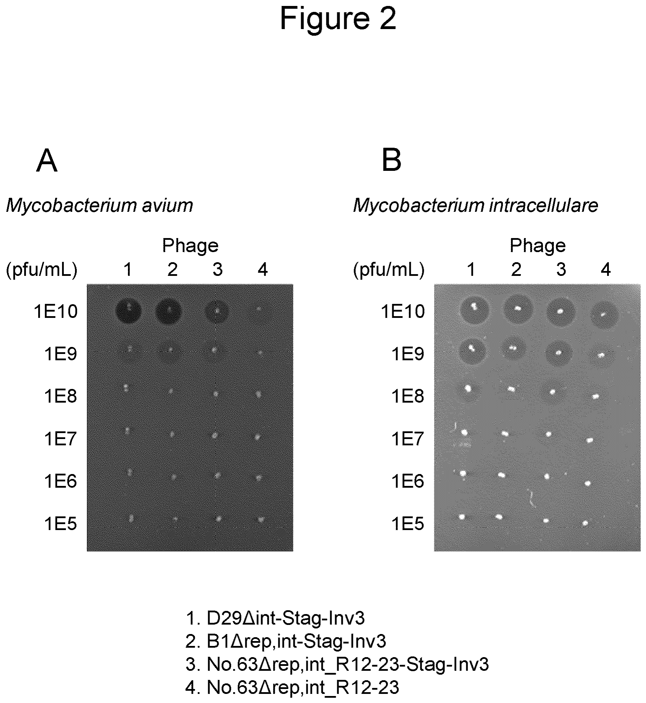

[Figure 2] Figure 2 shows photographs showing the lytic activity of 1. S-tag and Inv3 fusion D29 lysogenic gene-deficient strain (D29Δint-Stag-Inv3), 2. S-tag and Inv3 fusion B1 lysogenic gene-deficient strain (B1Δrep, int-Stag-Inv3), 3. S-tag and Inv3 fusion #63 freeze-thaw resistant strain (No. 63Δrep, int_R12-23-Stag-Inv3), and 4. #63 freeze-thaw resistant strain (No. 63Δrep, int_R12-23) against *Mycobacterium avium* KCH-ASGF-MA-05 (A) or clarithromycin-resistant *Mycobacterium intracellulare* KCH-ASGF-MAC-475 (B).

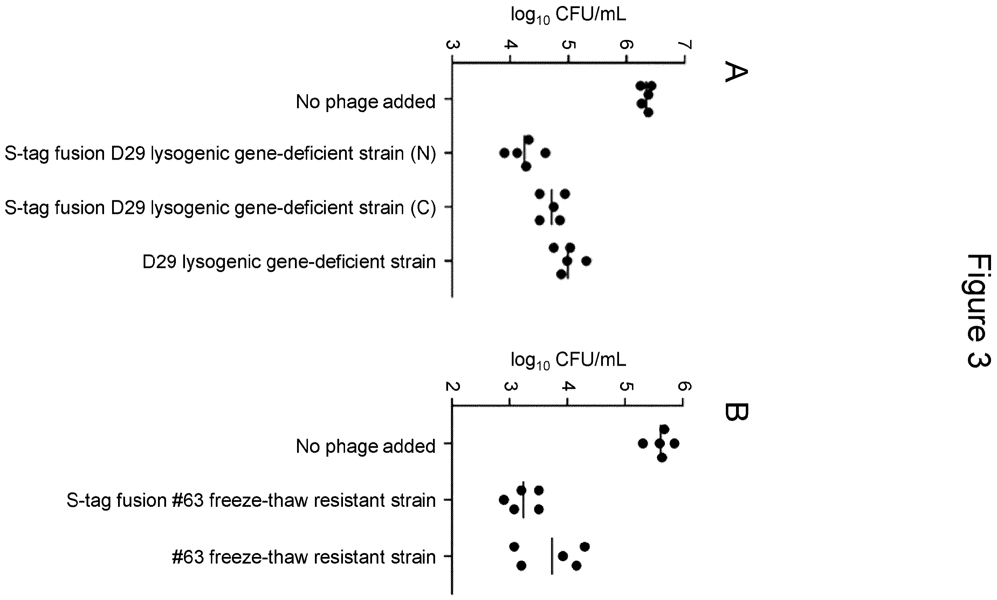

[Figure 3] Figure 3A is a graph showing the bactericidal activity of bacteriophages of S-tag fusion D29 lysogenic gene-deficient strain (N), S-tag fusion D29 lysogenic gene-deficient strain (C), and D29 lysogenic gene-deficient strain against intracellular *Mycobacterium avium* KCH-ASGF-MA-05. Figure 3B is a graph showing the bactericidal activity of bacteriophages of S-tag fusion #63 freeze-thaw resistant strain, and #63 freeze-thaw resistant strain against intracellular *Mycobacterium avium* KCH-ASGF-MA-12. The vertical axis indicates the colony forming unit (CFU/mL). Each bar indicates a geometric mean value.

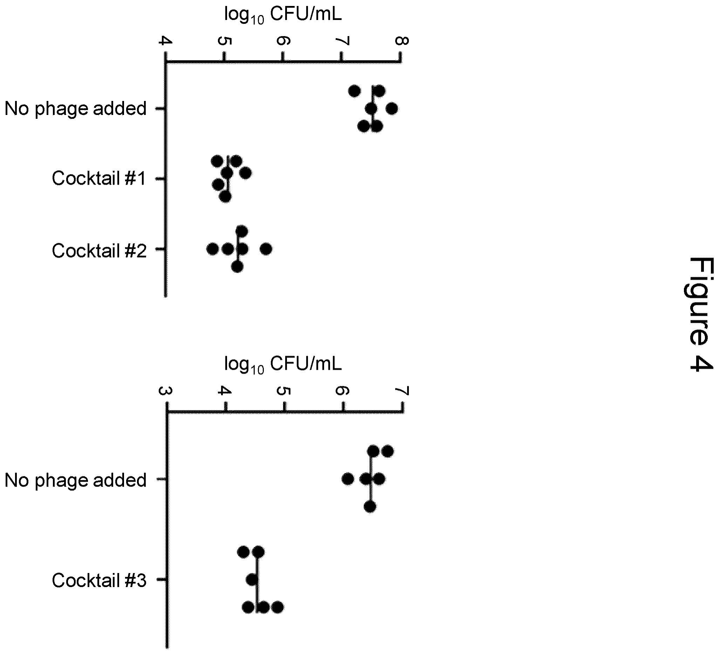

[Figure 4] Figure 4 are graphs showing the bactericidal activity of various bacteriophage cocktails against intracellular *Mycobacterium avium* KCH-ASGF-MA-05. The vertical axis indicates the colony forming unit (CFU/mL). Each bar indicates a geometric mean value.

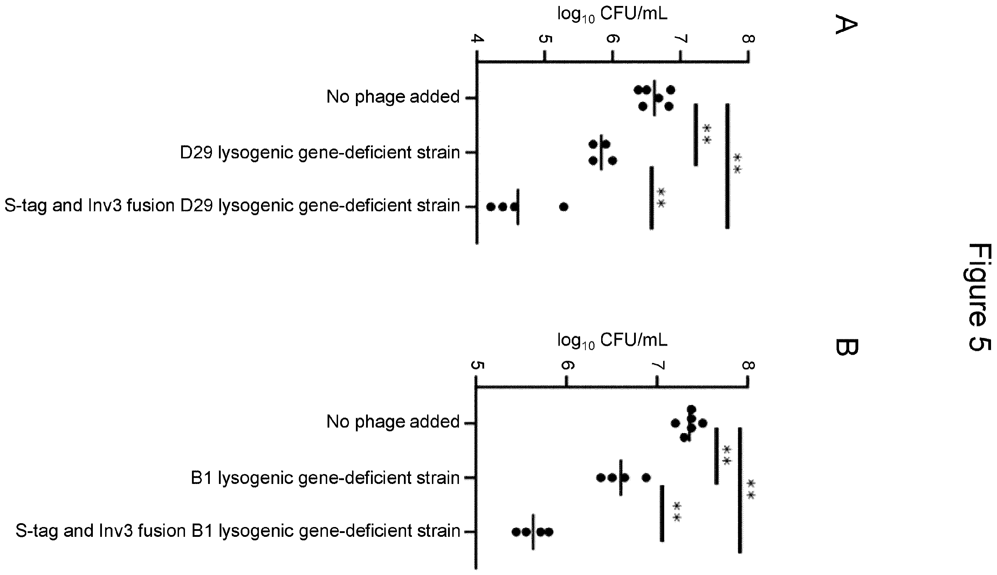

[Figure 5] Figure 5 are graphs showing the bactericidal activity of various bacteriophages against intracellular *Mycobacterium avium* KCH-ASGF-MA-05. The vertical axis indicates the colony forming unit (CFU/mL). Each bar indicates a geometric mean value. The symbol ** indicates that there was a significant difference between groups as determined by Tukey's multiple comparison test at a significance level of less than 1% (p < 0.01).

### Description of Embodiments

This application claims the priority of Japanese Patent Application No. 2023-116013, filed on July 14, 2023, the entire contents of which are incorporated herein by reference.

Hereinafter, the present invention will be described in detail. The following embodiments are examples for explaining the present invention and are not intended to limit the present invention only to these embodiments. The present invention can be embodied in various embodiments without departing from the gist thereof.

### < 1. Bacteriophage of the present invention >

In one embodiment, the present invention provides a new bacteriophage (hereinafter also referred to as the "bacteriophage of the present invention"). The bacteriophage of the present invention is a bacteriophage having lytic activity against non-tuberculous mycobacteria (NTM), particularly *Mycobacterium avium* and/or *Mycobacterium intracellulare,* which cause pulmonary MAC disease.

A #63 freeze-thaw resistant strain, which corresponds to the bacteriophage of the present invention, has been internationally deposited at the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the Applicant on June 22, 2023 (Accession No. NITE BP-03918).

In one embodiment, the bacteriophage of the present invention may include the following bacteriophage:
A bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03918; and (b) lacks a lysogenic gene.

The "identity" used herein refers to the identity of a nucleotide sequence or amino acid sequence determined by techniques known in the art. Generally available alignment software may be used as a method for sequence alignment. Examples include, but are not limited to, CLUSTAL W program (Nucleic Acids Research. 1994; 22 (22): 4673-80), FASTA program (Proceedings of the National Academy of Sciences of the United States of America. 1988; 85 (8): 2444-8), and BLAST programs (including BLASTP, BLASTN, BLASTX, TBLASTN, TBLASTX, etc.) (Journal of Molecular Biology. 1990; 215 (3): 403-10). It means a value Identity obtained by a specific method for sequence alignment using parameters prepared by default by, for example, NEEDLE program (Journal of Molecular Biology. 1970; 48 (3): 443-53) search. The parameters are as follows.
Gap penalty = 10
Extend penalty = 0.5
Matrix = EBLOSUM62

The "lysogenic gene(s)" used herein means a gene(s) involved in the induction and/or maintenance of a lysogenic state. Examples of the "lysogenic gene(s)" may include integrase genes, immunity repressor genes, and Cro genes. Specifically, lysogenic genes and their sequence information are also known (Nat Med. 2019; 25 (5): 730.; mBio. 2021; 12 (3): e00973.; Cell. 2018; 172 (6): 1260.).

As used herein, "lacking a lysogenic gene" means that: 1) the full-length of at least one lysogenic gene is lacking from the sequence of the genome of the bacteriophage; or 2-1) the bacteriophage becomes a lytic bacteriophage by modifying a part of at least one lysogenic gene region by deletion, substitution, insertion, addition, or a combination thereof from the sequence of the genome of the bacteriophage; or 2-2) the function of the lysogenic gene is lacking. The lysogenic gene can be deleted by a known method in the art depending on the type of the lysogenic gene to be deleted (e.g., Nat Med. 2019; 25 (5): 730.; mBio. 2021; 12 (3): e00973.; PLoS One. 2008; 3 (12): e3957.).

As used herein, "having lytic activity against *Mycobacterium avium* and/or *Mycobacterium intracellulare"* means to show lytic plaques against at least one strain of bacteria belonging to *Mycobacterium avium* or *Mycobacterium intracellulare.* Confirmation of lytic activity against these bacteria can be performed using a method and means known in the art. For example, whether or not a bacteriophage has lytic activity can be confirmed using the method described herein in Example 4 by confirming whether or not about 10¹⁰ pfu/mL to 10⁵ pfu/mL of bacteriophage (or mixture of a plurality of bacteriophages) exhibits lytic plaques *against Mycobacterium avium* or *Mycobacterium intracellulare.*

In one embodiment, the bacteriophage of the present invention may be resistant to freeze-thaw. For example, the bacteriophage of the present invention may include the following bacteriophage:
A bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918; and (b) lacks a lysogenic gene, and
the bacteriophage is resistant to freeze-thaw.

Herein, the phrase "resistant to freeze-thaw" means that the bacteriophage is maintained at a desired level even after a freeze-thaw treatment although it is known that a part of a bacteriophage may be killed or damaged by a freeze-thaw treatment. For example, the number of bacteriophages after a freeze-thaw treatment is maintained at a level substantially equivalent to that before the freeze-thaw treatment, or is maintained at at least 10%, at least 20%, at least 30%, at least 40%, at least 50%, at least 60%, at least 70%, at least 80%, or at least 90% or more compared to that before the freeze-thaw treatment. Whether or not a bacteriophage is resistant to freeze-thaw can be confirmed by methods and means known in the art. For example, whether a bacteriophage is resistant to freeze-thaw can be confirmed using a method described herein in Example 2 to check whether or not the bacteriophage shows a reduction in count after the freeze-thaw treatment. Alternatively, whether or not a bacteriophage is resistant to freeze-thaw can also be confirmed by checking whether or not the activity of the bacteriophage (for example, the lytic activity against *Mycobacterium avium* and/or *Mycobacterium intracellulare*) is maintained at a desired level after the freeze-thaw treatment. For example, when the bacteriophage maintains the activity at a level substantially equivalent to that before the freeze-thaw treatment, or maintains the activity at at least 10%, at least 20%, at least 30%, at least 40%, at least 50%, at least 60%, at least 70%, at least 80%, or at least 90% or more compared to that before the freeze-thaw treatment, the bacteriophage is considered to be resistant to freeze-thaw.

In one embodiment, the bacteriophage of the present invention may include the following bacteriophage:
a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03918; and (b) lacks an integrase gene and/or an immunity repressor gene.

The phrase "lacking an integrase gene and/or an immunity repressor gene" means: 1) the full-length of the integrase gene and/or the immunity repressor gene is deleted from the sequence of the genome of the bacteriophage; or 2-1) the bacteriophage becomes a lytic bacteriophage by modifying a part of the integrase gene and/or the immunity repressor gene from the sequence of the genome of the bacteriophage by deletion, substitution, insertion, addition, or a combination thereof; or 2-2) the function of the integrase gene and/or the immunity repressor gene is deficient. As mentioned above, a lysogenic gene such as an integrase gene and an immunity repressor gene can be deleted by a method known in the art depending on the type of the lysogenic gene to be deleted (e.g., Nat Med. 2019; 25 (5): 730.; mBio. 2021; 12 (3): e00973.; PLoS One. 2008; 3 (12): e3957.).

In one embodiment, the bacteriophage of the present invention may include a bacteriophage having lytic activity against *Mycobacterium avium* and/or *Mycobacterium intracellulare,* wherein a genome of the bacteriophage: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03918; and (b) lacks an integrase gene and/or an immunity repressor gene.

The bacteriophage of the present invention may include the following bacteriophage:
a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918.

The bacteriophage of the present invention may include the following bacteriophage:
a bacteriophage wherein a genome of the bacteriophage consists of a nucleic acid sequence of a genome of the bacteriophage identified by Accession No. NITE BP-03918.

The bacteriophage of the present invention may also include the following bacteriophage:
the bacteriophage identified by Accession No. NITE BP-03918.

The bacteriophage of the present invention may also include the following bacteriophage:
a bacteriophage having lytic activity against *Mycobacterium avium* and/or *Mycobacterium intracellulare,* wherein a genome of the bacteriophage: (a) comprises a nucleic acid sequence modified from a nucleic acid sequence of a genome of the bacteriophage identified by Accession No. NITE BP-03918 by deletion, substitution, insertion, or addition of 1 to 5000 (e.g., 1 to 1000, 1 to 500, or 1 to 100) nucleotides, or a combination thereof; and (b) lacks an integrase gene and/or an immunity repressor gene.

For the modifications such as deletion, substitution, insertion, or addition of a nucleotide(s), a plurality of modifications may be continuous, or a plurality of modifications may exist at different positions. Such a modified form of the bacteriophage is encompassed in the bacteriophage of the present invention as long as it has lytic activity against *Mycobacterium avium* and/or *Mycobacterium intracellulare* (and optionally is resistant to freeze-thaw).

During passage culture, production, and/or replication of the bacteriophage, bacteriophage mutants in which the nucleic acid sequences of the genomes contained in the bacteriophage are partially deleted, substituted, inserted, and/or added may occur. Such mutants may be included in the bacteriophage of the present invention, as long as they have lytic activity against *Mycobacterium avium* and/or *Mycobacterium intracellulare* (and optionally are resistant to freeze-thaw).

The bacteriophage of the present invention also includes a passage strain of a bacteriophage identified by above-described Accession No. NITE BP-03918, as long as it has lytic activity against *Mycobacterium avium* and/or *Mycobacterium intracellulare* (and optionally is resistant to freeze-thaw).

As used herein, the term "passage strain" means a bacteriophage obtained by passage culture of a distributed bacteriophage.

The present invention may also include a polynucleotide consisting of the genome contained in the bacteriophage of the present invention (hereinafter referred to as "genome of the present invention"). Accordingly, the present invention provides: a polynucleotide of a bacteriophage genome that comprises a nucleic acid sequence having 90% or more identity to the nucleic acid sequence of the genome of a bacteriophage identified by Accession No. NITE BP-03918 and lacks an integrase gene and/or an immunity repressor gene; and a polynucleotide of a bacteriophage genome that comprises a nucleic acid sequence having 90% or more identity to the nucleic acid sequence of the genome of a bacteriophage identified by Accession No. NITE BP-03918. In one embodiment, the polynucleotide may be a polynucleotide encoding a bacteriophage having lytic activity against *Mycobacterium avium* and/or *Mycobacterium*

### intracellulare.

In one embodiment, the genome of the present invention may include a genome that: (a) comprises a nucleic acid sequence having 90% or more, 95% or more, 96% or more, 97% or more, 98% or more, or 99% or more identity to a nucleic acid sequence of a genome of the bacteriophage identified by Accession No. NITE BP-03918; and (b) lacks an integrase gene and/or an immunity repressor gene.

The genome of the present invention may include the following genome:
a genome that comprises a nucleic acid sequence of a genome of the bacteriophage identified by Accession No. NITE BP-03918.

The genome of the present invention may include the following genome:
a genome consisting of a nucleic acid sequence of a genome of the bacteriophage identified by Accession No. NITE BP-03918.

The genome of the present invention can be produced using conventional techniques known in the art, such as recombinant DNA technology (e.g., polymerase chain reaction (PCR) amplification, cloning), enzymatic or chemical synthesis, or a combination thereof. For example, the genome of the present invention can be produced by ligating a plurality of polynucleotides containing the partial nucleotide sequences of the genome of the present invention by genetic engineering techniques. In one embodiment, the full-length of or partial sequence of the genome of the present invention may be contained in a vector known in the art.

The bacteriophage of the present invention can be obtained by making a request for distribution to the depository center.

Further, the bacteriophage of the present invention can be produced based on the sequence information by sequencing the nucleic acid sequence of the genome of the distributed bacteriophage by general techniques known in the art. For example, the genome of the present invention produced by the above method may be introduced into a host bacterium by electroporation (e.g., in the case of obtaining a bacteriophage having lytic activity against the genus *Mycobacterium,* the genus *Mycobacterium* may be used as the host bacterium). Then, the bacterium into which the genome has been introduced may be added to a plate overlaid with soft agar and cultured. Thereafter, single lytic plaques may be obtained by plaque assay. The bacteriophage of the present invention can be produced by adding the single lytic plaques to the host bacterial culture solution, followed by culturing and filtering the culture supernatant obtained by standing or centrifugation. Further, in order to produce the bacteriophage of the present invention, a lysogenic gene can be deleted from the phage genome using Bacteriophage Recombineering of Electroporated DNA (BRED) (PLoS One. 2008; 3 (12): e3957.).

The bacteriophage of the present invention can be prepared by conventional culture, isolation, and purification methods known in the art. For example, host bacteria (such as *Mycobacterium smegmatis, Mycobacterium avium,* or *Mycobacterium intracellulare*) may be pre-cultured, infected with the bacteriophage of the present invention, and cultured at 37°C. After culturing, the culture supernatant obtained by standing or centrifugation can be filtered, to obtain a purified bacteriophage. The medium can be appropriately selected depending on the bacterium to be used. For example, in the case of culturing *Mycobacterium smegmatis,* 7H9 medium can be used. In the case of preparing a plurality of bacteriophages, they may be grown in separate host bacteria, or they may be grown in the same host bacterium.

Further, the bacteriophage of the present invention can be stored in various forms (such as in liquid and freeze-dried products) by an appropriate method known in the art.

### < 2. Modified bacteriophage of the present invention >

In another aspect, the present invention provides a modified bacteriophage (hereinafter also referred to as the "modified bacteriophage of the present invention"). The modified bacteriophage of the present invention is a bacteriophage in which the head (also referred to as the capsid) and/or the tail is modified.

A bacteriophage to be modified is not especially limited as long as it is a bacteriophage having lytic activity against non-tuberculous mycobacteria (NTM), particularly *Mycobacterium avium* and/or *Mycobacterium intracellulare,* which cause pulmonary MAC disease. For example, any one of bacteriophages of the following (1) to (3) can be modified:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513, and (b) lacks a lysogenic gene;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03918, and (b) lacks a lysogenic gene; and
(3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514, and (b) lacks a lysogenic gene.

In one embodiment, the bacteriophage to be modified includes any one of the following (1) to (3):
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513, and (b) lacks an integrase gene and/or an immunity repressor gene;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene; and
(3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514, and (b) lacks an integrase gene and/or an immunity repressor gene.

In one embodiment, the bacteriophage to be modified includes any one of the following (1) to (3):
(1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513;
(2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918; and
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514.

In one embodiment, the bacteriophage to be modified includes any one of the following (1) to (3):
(1) a bacteriophage wherein a genome of the bacteriophage consists of a nucleic acid sequence of a genome of the bacteriophage identified by Accession No. NITE BP-03513;
(2) a bacteriophage wherein a genome of the bacteriophage consists of a nucleic acid sequence of a genome of the bacteriophage identified by Accession No. NITE BP-03918; and
(3) a bacteriophage wherein a genome of the bacteriophage consists of a nucleic acid sequence of a genome of the bacteriophage identified by Accession No. NITE BP-03514.

The bacteriophage to be modified also includes a passage strain of a bacteriophage identified by any of above-described Accession Nos. NITE BP-03513, NITE BP-03918, and NITE BP-03514 as long as it has lytic activity against *Mycobacterium avium* and/or *Mycobacterium intracellulare.*

In one embodiment, the bacteriophage to be modified includes any one of the following (1) to (3):
(1) a bacteriophage identified by Accession No. NITE BP-03513 or a passage strain thereof;
(2) a bacteriophage identified by Accession No. NITE BP-03918 or a passage strain thereof; and
(3) a bacteriophage identified by Accession No. NITE BP-03514 or a passage strain thereof.

The bacteriophage identified by Accession No. NITE BP-03918 (#63 freeze-thaw resistant strain #63 ΔR12-23) has been internationally deposited at the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the Applicant on June 22, 2023 (Accession No. NITE BP-03918).

The bacteriophage identified by Accession No. NITE BP-03513 (D29 lysogenic gene-deficient strain D29Δ) has been internationally deposited at the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the Applicant on August 26, 2021 (Accession No. NITE BP-03513).

The bacteriophage identified by Accession No. NITE BP-03514 (B1 lysogenic gene-deficient strain B1Δ) has been internationally deposited at the National Institute of Technology and Evaluation, Patent Microorganisms Depositary (Kazusakamatari 2-5-8 122, Kisarazu, Chiba 292-0818 Japan) by the Applicant on August 26, 2021 (Accession No. NITE BP-03514).

For bacteriophages to be modified, a bacteriophage identified by Accession Number can be obtained by making a request for distribution to the Depository.

The bacteriophage to be modified can be prepared and cultured in the same manner as described in the preceding section < 1. Bacteriophage of the present invention >.

In one embodiment, the modification includes linkage of the capsid and/or the tail to a tag peptide. Various tag peptides known in the art, such as an S-tag, a T7-tag, a His-tag, and a FLAG tag, may be used as the tag peptide. In one embodiment, the modification includes linkage of the capsid and/or the tail to an S-tag. Specifically, the capsid and/or the tail of the modified bacteriophage of the present invention is linked to an S-tag. In one embodiment, the modification includes linkage of the capsid with an S-tag.

S-tag is a kind of tag peptide, contains an amino acid sequence WSHPQFEK (SEQ ID NO: 2), and is known in the art. The number of S-tags linked to the capsid and/or tail is not especially limited, and for example, may be one, two, or four per arbitrary protein.

As a method for linking a tag peptide (such as an S-tag) to the capsid and/or the tail of a bacteriophage, a person skilled in the art can select any of conventional methods. For example, a bacteriophage having an S-tag linked to the capsid and/or tail thereof can be produced by introducing, into a host bacterium of the bacteriophage, a plasmid in which a polynucleotide containing a DNA sequence encoding the S-tag peptide is linked upstream or downstream of a DNA sequence encoding a capsid structural protein and/or tail structural protein, expressing a fusion protein of the capsid structural protein and the S-tag in the host bacterium, and subsequently infecting the resultant with the bacteriophage. Herein, the bacteriophage having an S-tag linked to the capsid and/or the tail thereof is sometimes referred to as the S-tag fusion bacteriophage. As the capsid structural protein and/or the tail structural protein, it is possible to use proteins generally known to constitute the capsid, such as a major capsid protein, a minor capsid protein, an accessory protein, and a neck protein, or proteins belonging to a protein group designated as tail proteins that are known to constitute the tail. For example, a modified phage having an S-tag linked to the capsid is reported in Non Patent Literature 1. The linkage of an S-tag to the capsid may be a linkage of an S-tag peptide to the N-terminal side of the capsid structural protein or to the C-terminal side thereof.

The modified bacteriophage having a tag peptide (e.g., an S-tag) linked to the capsid and/or the tail thereof exhibits higher lytic activity against *Mycobacterium avium* and/or *Mycobacterium intracellulare* compared to an unmodified bacteriophage. For example, the modified bacteriophage has lytic activity higher by at least 5%, at least 10%, at least 20%, at least 30%, at least 40%, or at least 50% compared to the unmodified bacteriophage. The lytic activity can be confirmed by, for example, a method described in Example 5.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03513, and (b) lacks an integrase gene and/or an immunity repressor gene, and
the capsid of the bacteriophage is linked to an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513, and the capsid of the bacteriophage is linked to an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to an S-tag.

In one embodiment, the modified bacteriophage of the present invention includes the following:
a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to an S-tag.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene, and
the capsid of the bacteriophage is linked to an S-tag.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene,
the bacteriophage possesses resistance to freeze-thaw, and
the capsid of the bacteriophage is linked to an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918, and the capsid of the bacteriophage is linked to an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, and the capsid of the bacteriophage is linked to an S-tag.

In one embodiment, the modified bacteriophage of the present invention includes the following:
a bacteriophage having lytic activity against *Mycobacterium avium* and/or *Mycobacterium intracellulare,* wherein the bacteriophage is a bacteriophage identified by Accession No. NITE BP-03918 or a passage strain thereof, and the capsid of the bacteriophage or the passage strain thereof is linked to an S-tag.

In one embodiment, the modification includes direct or indirect linkage of the capsid and/or the tail to a cell-penetrating peptide. Specifically, the capsid and/or the tail of the modified bacteriophage of the present invention is directly or indirectly linked to a cell-penetrating peptide. For indirect linkage, for example, a linker or a tag can be used.

In one embodiment, the modification includes direct linkage of the capsid and/or the tail to a cell-penetrating peptide.

Herein, a cell-penetrating peptide (CPP) means a peptide that, when linked to the capsid and/or the tail of a bacteriophage, increases the intracellular uptake of that bacteriophage. CPPs are known in the art, and the sequences and mechanisms of a large number of CPPs have been reported (for example, Crit. Rev. Microbiol. 2021; 47 (4): 461). Examples of the CPP include, but are not limited to, Inv3 peptide (TKRRITPKDVIDVRSVTTEINT; SEQ ID NO: 1) corresponding to residues 25 to 46 and Inv5 peptide corresponding to residues 1 to 60 of the rMce1a sequence (Anal. Biochem. 2006; 353 (1): 7); and R9, TAT, TP10, and L17E.

In one embodiment, the modification includes linkage of the capsid and/or the tail to a cell-penetrating peptide via a tag.

The tag is not especially limited as long as it is a tag peptide capable of linking the capsid and/or the tail to a cell-penetrating peptide. Examples of the tag include an S-tag, a T7-tag, a His-tag, and a FLAG tag. The linkage of the capsid and/or the tail to a cell-penetrating peptide via a tag may be a linkage to the N-terminal side of the capsid structural protein and/or the tail structural protein, or to the C-terminal side thereof.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03513, and (b) lacks an integrase gene and/or an immunity repressor gene, and
the capsid of the bacteriophage is linked to a cell-penetrating peptide via an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513, and the capsid of the bacteriophage is linked to a cell-penetrating peptide via an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to a cell-penetrating peptide via an S-tag.

In one embodiment, the modified bacteriophage of the present invention includes the following:
a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to a cell-penetrating peptide via an S-tag.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene, and
the capsid of the bacteriophage is linked to a cell-penetrating peptide.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene,
the bacteriophage possesses resistance to freeze-thaw, and
the capsid of the bacteriophage is linked to a cell-penetrating peptide.

In one embodiment, the modified bacteriophage of the present invention includes the following:
a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918, and the capsid of the bacteriophage is linked to a cell-penetrating peptide.

In one embodiment, the modified bacteriophage of the present invention includes the following:
a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, and the capsid of the bacteriophage is linked to a cell-penetrating peptide.

In one embodiment, the modified bacteriophage of the present invention includes the following:
a bacteriophage having lytic activity against *Mycobacterium avium* and/or *Mycobacterium intracellulare,* wherein the bacteriophage is a bacteriophage identified by Accession No. NITE BP-03918 or a passage strain thereof, and the capsid of the bacteriophage or the passage strain thereof is linked to a cell-penetrating peptide.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene, and
the capsid of the bacteriophage is linked to a cell-penetrating peptide via a tag.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene,
the bacteriophage possesses resistance to freeze-thaw, and
the capsid of the bacteriophage is linked to a cell-penetrating peptide via a tag.

In one embodiment, the modified bacteriophage of the present invention includes the following:
a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918, and the capsid of the bacteriophage is linked to a cell-penetrating peptide via a tag.

In one embodiment, the modified bacteriophage of the present invention includes the following:
a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, and the capsid of the bacteriophage is linked to a cell-penetrating peptide via a tag.

In one embodiment, the modified bacteriophage of the present invention includes the following:
a bacteriophage having lytic activity against *Mycobacterium avium* and/or *Mycobacterium intracellulare,* wherein the bacteriophage is a bacteriophage identified by Accession No. NITE BP-03918 or a passage strain thereof, and the capsid of the bacteriophage or the passage strain thereof is linked to a cell-penetrating peptide via a tag.

In one embodiment, the tag linked to the modified bacteriophage includes an S-tag.

In one embodiment, the cell-penetrating peptide linked to the modified bacteriophage includes Inv3.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03513, and (b) lacks an integrase gene and/or an immunity repressor gene, and
the capsid of the bacteriophage is linked to Inv3 via an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513, and the capsid of the bacteriophage is linked to Inv3 via an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to Inv3 via an S-tag.

In one embodiment, the modified bacteriophage of the present invention includes the following:
a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to Inv3 via an S-tag.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene, and
the capsid of the bacteriophage is linked to Inv3 via an S-tag.

In one embodiment, the modified bacteriophage of the present invention includes the following:
a bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene,
the bacteriophage possesses resistance to freeze-thaw, and
the capsid of the bacteriophage is linked to Inv3 via an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918, and the capsid of the bacteriophage is linked to Inv3 via an S-tag.

In one embodiment, the modified bacteriophage of the present invention includes the following:
a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, and the capsid of the bacteriophage is linked to Inv3 via an S-tag.

In one embodiment, the modified bacteriophage of the present invention includes the following:
a bacteriophage having lytic activity against *Mycobacterium avium* and/or *Mycobacterium intracellulare,* wherein the bacteriophage is a bacteriophage identified by Accession No. NITE BP-03918 or a passage strain thereof, and the capsid of the bacteriophage or the passage strain thereof is linked to Inv3 via an S-tag.

A person skilled in the art can link a cell-penetrating peptide to the capsid and/or the tail directly or via a tag by any conventional method (Non Patent Literature 1 and Non Patent Literature 2). For example, a bacteriophage having a cell-penetrating peptide, or a tag and a cell-penetrating peptide linked to the capsid and/or tail thereof can be produced by introducing a plasmid, in which a polynucleotide containing a DNA sequence, encoding the cell-penetrating peptide or the tag and the cell-penetrating peptide, is linked upstream or downstream of a DNA sequence encoding a capsid structural protein and/or tail structural protein, into a host bacterium of the bacteriophage; expressing, in the host bacterium, a fusion protein of the capsid structural protein and/or the tail structural protein with the cell-penetrating peptide, or the tag and the cell-penetrating peptide; and subsequently infecting the resultant with the bacteriophage. Herein, the bacteriophage having a tag and a cell-penetrating peptide linked to the capsid and/or the tail is sometimes referred to as the tag and cell-penetrating peptide fusion bacteriophage. As the capsid structural protein and/or the tail structural protein, it is possible to use proteins generally known to constitute the capsid, such as a major capsid protein, a minor capsid protein, an accessory protein, and a neck protein, or proteins belonging to a protein group designated as tail proteins that are known to constitute the tail.

The modified bacteriophage having a cell-penetrating peptide linked to the capsid and/or the tail directly or via a tag has higher lytic activity against intracellular *Mycobacterium avium* and/or *Mycobacterium intracellulare* compared to an unmodified bacteriophage. For example, the modified bacteriophage has lytic activity against intracellular *Mycobacterium avium* and/or *Mycobacterium intracellulare* higher by at least 5%, at least 10%, at least 20%, at least 30%, at least 40%, or at least 50% compared to the unmodified bacteriophage. The lytic activity against intracellular *Mycobacterium avium* and/or *Mycobacterium intracellulare* can be confirmed by, for example, checking whether or not the bacteriophage shows a reduction in count of *Mycobacterium avium* and/or *Mycobacterium intracellulare* after the treatment with the modified bacteriophage (or a mixture of a plurality of bacteriophages) by a method described in Examples 5 to 6.

### < 3. Uses of pharmaceutical composition and bacteriophage of the present invention >

The bacteriophage of the present invention and/or the modified bacteriophage of the present invention have lytic activity against non-tuberculous mycobacteria (NTM), particularly *Mycobacterium avium* and/or *Mycobacterium intracellulare,* which cause pulmonary MAC disease. Therefore, the bacteriophage of the present invention and/or the modified bacteriophage of the present invention are expected to be effective for treating or preventing nontuberculous mycobacterial disease (NTM disease), particularly pulmonary MAC disease.

In another aspect, the present invention provides uses of the bacteriophage of the present invention and/or the modified bacteriophage of the present invention, such as a pharmaceutical composition comprising the bacteriophage of the present invention and/or the modified bacteriophage of the present invention as an active component (which may be hereinafter referred to as "pharmaceutical composition of the present invention").

The pharmaceutical composition of the present invention may include a pharmaceutical composition comprising at least one bacteriophage including the bacteriophage of the present invention and/or the modified bacteriophage of the present invention, and a pharmaceutically acceptable excipient.

The pharmaceutical composition of the present invention may further comprise one or more strains of a bacteriophage known to have lytic activity against *Mycobacterium avium* and/or *Mycobacterium intracellulare.* Examples of such a strain include, but are not limited to, the bacteriophage of the present invention, the modified bacteriophage of the present invention, and a bacteriophage described in International Application No. PCT/JP2023/016600.

In one embodiment, the pharmaceutical composition of the present invention may comprise the following modified bacteriophage of the present invention: a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513, and (b) lacks an integrase gene and/or an immunity repressor gene, and the capsid of the bacteriophage is linked to a cell-penetrating peptide via an S-tag; and
one or more bacteriophages selected from the group consisting of bacteriophages of the following (1) to (3):
(1) (A) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514, and (b) lacks an integrase gene and/or an immunity repressor gene, or (B) the bacteriophage (A) in which the capsid of the bacteriophage is linked to a cell-penetrating peptide via a tag;
(2) (C) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene, or (D) the bacteriophage (C) in which the capsid of the bacteriophage is linked to a cell-penetrating peptide via a tag; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519.

The bacteriophage identified by Accession No. NITE BP-03519 (#123 parent strain) has been internationally deposited at the National Institute of Technology and Evaluation, Patent Microorganisms Depositary (Kazusakamatari 2-5-8 122, Kisarazu, Chiba 292-0818 Japan) that is the International Depositary Authority based on the provisions of the Budapest Treaty for the Deposit of Patented Microorganisms by the Applicant on August 26, 2021 (Accession No. NITE BP-03519).

In one embodiment, the pharmaceutical composition of the present invention may comprise the following modified bacteriophage of the present invention: a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513, and the capsid of the bacteriophage is linked to an S-tag; and
one or more bacteriophages selected from the group consisting of bacteriophages of the following (1) to (3):
(1) (A) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514, or (B) the bacteriophage (A) in which the capsid of the bacteriophage is linked to a cell-penetrating peptide via a tag;
(2) (C) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918, or (D) the bacteriophage (C) in which the capsid of the bacteriophage is linked to a cell-penetrating peptide via a tag; and
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519.

In one embodiment, the pharmaceutical composition of the present invention may comprise the following modified bacteriophage of the present invention: a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to an S-tag; and
one or more bacteriophages selected from the group consisting of bacteriophages of the following (1) to (3):
(1) (A) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03514, or (B) the bacteriophage (A) in which the capsid of the bacteriophage is linked to a cell-penetrating peptide via a tag;
(2) (C) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, or (D) the bacteriophage (C) in which the capsid of the bacteriophage is linked to a cell-penetrating peptide via a tag; and
(3) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03519.

In one embodiment, the pharmaceutical composition of the present invention may comprise the following modified bacteriophage of the present invention: a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to an S-tag; and
one or more bacteriophages selected from the group consisting of bacteriophages of the following (1) to (3):
(1) (A) a bacteriophage having lytic activity against *Mycobacterium avium* and/or *Mycobacterium intracellulare,* wherein the bacteriophage is a bacteriophage identified by Accession No. NITE BP-03514 or a passage strain thereof, or (B) the bacteriophage (A) in which the capsid of the bacteriophage or the passage strain thereof is linked to a cell-penetrating peptide via a tag;
(2) (C) a bacteriophage having lytic activity against *Mycobacterium avium* and/or *Mycobacterium intracellulare,* wherein the bacteriophage is a bacteriophage identified by Accession No. NITE BP-03918 or a passage strain thereof, or (D) the bacteriophage (C) in which the capsid of the bacteriophage or the passage strain thereof is linked to a cell-penetrating peptide via a tag; and
(3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.

In one embodiment, the pharmaceutical composition of the present invention may comprise the following modified bacteriophage of the present invention: a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to Inv3 via an S-tag; and
one or more bacteriophages selected from the group consisting of bacteriophages of the following (1) to (3):
(1) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03514;
(2) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, and the capsid of the bacteriophage is linked to Inv3 via an S-tag; and
(3) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03519.

In one embodiment, the pharmaceutical composition of the present invention may comprise the following modified bacteriophage of the present invention: a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to Inv3 via an S-tag; and
one or more bacteriophages selected from the group consisting of bacteriophages of the following (1) to (3):
(1) a bacteriophage having lytic activity against *Mycobacterium avium* and/or *Mycobacterium intracellulare,* wherein the bacteriophage is a bacteriophage identified by Accession No. NITE BP-03514 or a passage strain thereof;
(2) a bacteriophage having lytic activity against *Mycobacterium avium* and/or *Mycobacterium intracellulare,* wherein the bacteriophage is a bacteriophage identified by Accession No. NITE BP-03918 or a passage strain thereof, and the capsid of the bacteriophage or the passage strain thereof is linked to Inv3 via an S-tag; and
(3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.

In one embodiment, the pharmaceutical composition of the present invention may comprise the following modified bacteriophage of the present invention: a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, and the capsid of the bacteriophage is linked to Inv3 via an S-tag; and
one or more bacteriophages selected from the group consisting of bacteriophages of the following (1) to (3):
(1) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03514; and
(3) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03519.

In one embodiment, the pharmaceutical composition of the present invention may comprise the following modified bacteriophage of the present invention: a bacteriophage having lytic activity against *Mycobacterium avium* and/or *Mycobacterium intracellulare,* wherein the bacteriophage is a bacteriophage identified by Accession No. NITE BP-03918 or a passage strain thereof, and the capsid of the bacteriophage or the passage strain thereof is linked to Inv3 via an S-tag; and
one or more bacteriophages selected from the group consisting of bacteriophages of the following (1) to (3):
(1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to Inv3 via an S-tag;
(2) a bacteriophage having lytic activity against *Mycobacterium avium* and/or *Mycobacterium intracellulare,* wherein the bacteriophage is a bacteriophage identified by Accession No. NITE BP-03514 or a passage strain thereof; and
(3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.

The pharmaceutical composition of the present invention can be prepared using an excipient conventionally used in the art, that is, a pharmaceutical excipient, a pharmaceutical carrier, and the like by a conventional method. Examples of dosage forms of such a pharmaceutical composition may include parenteral formulations such as injections, infusions, powder inhalers, and nebulizers, and it can be administered by intravenous administration, transpulmonary administration, or the like. For formulation, excipients, carriers, additives, or the like can be used depending on these dosage forms within a pharmaceutically acceptable range. For example, the pharmaceutical composition of the present invention can be produced by mixing the bacteriophage with a pharmaceutically acceptable excipient or suspending the bacteriophage in a pharmaceutically acceptable excipient.

The pharmaceutical composition of the present invention may include a pharmaceutical composition comprising at least two bacteriophages and a pharmaceutically acceptable excipient.

The pharmaceutical composition of the present invention may include a pharmaceutical composition comprising three bacteriophages and a pharmaceutically acceptable excipient.

In one embodiment, the pharmaceutical composition of the present invention may include a pharmaceutical composition comprising bacteriophages of the following (1) to (3) and a pharmaceutically acceptable excipient:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513, and (b) lacks an integrase gene and/or an immunity repressor gene, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514, and (b) lacks an integrase gene and/or an immunity repressor gene; and
(3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene, and the capsid of the bacteriophage is linked to Inv3 via an S-tag.

In the above-described embodiment, the pharmaceutical composition of the present invention comprises bacteriophages of the following (1) to (3):
(1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514; and
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918, and the capsid of the bacteriophage is linked to Inv3 via an S-tag.

In the above-described embodiment, the pharmaceutical composition of the present invention comprises bacteriophages of the following (1) to (3):
(1) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03514; and
(3) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, and the capsid of the bacteriophage is linked to Inv3 via an S-tag.

In one embodiment, the pharmaceutical composition of the present invention may include a pharmaceutical composition comprising bacteriophages of the following (1) to (3) and a pharmaceutically acceptable excipient:
(1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to Inv3 via an S-tag;
(2) a bacteriophage having lytic activity against *Mycobacterium avium* and/or *Mycobacterium intracellulare,* wherein the bacteriophage is a bacteriophage identified by Accession No. NITE BP-03514 or a passage strain thereof; and
(3) a bacteriophage having lytic activity against *Mycobacterium avium* and/or *Mycobacterium intracellulare,* wherein the bacteriophage is a bacteriophage identified by Accession No. NITE BP-03918 or a passage strain thereof, and the capsid of the bacteriophage or the passage strain thereof is linked to Inv3 via an S-tag.

In one embodiment, the pharmaceutical composition of the present invention may include a pharmaceutical composition comprising bacteriophages of the following (1) to (3) and a pharmaceutically acceptable excipient:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513, and (b) lacks an integrase gene and/or an immunity repressor gene, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03918, and (b) lacks an integrase gene and/or an immunity repressor gene, and the capsid of the bacteriophage is linked to Inv3 via an S-tag; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519.

In the above-described embodiment, the pharmaceutical composition of the present invention comprises bacteriophages of the following (1) to (3):
(1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03918, and the capsid of the bacteriophage is linked to Inv3 via an S-tag; and
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519.

In the above-described embodiment, the pharmaceutical composition of the present invention comprises bacteriophages of the following (1) to (3):
(1) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03918, and the capsid of the bacteriophage is linked to Inv3 via an S-tag; and
(3) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03519.

In one embodiment, the pharmaceutical composition of the present invention may include a pharmaceutical composition comprising bacteriophages of the following (1) to (3) and a pharmaceutically acceptable excipient:
(1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to Inv3 via an S-tag;
(2) a bacteriophage having lytic activity against *Mycobacterium avium* and/or *Mycobacterium intracellulare,* wherein the bacteriophage is a bacteriophage identified by Accession No. NITE BP-03918 or a passage strain thereof, and the capsid of the bacteriophage or the passage strain thereof is linked to Inv3 via an S-tag; and
(3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.

In one embodiment, the pharmaceutical composition of the present invention may include a pharmaceutical composition comprising bacteriophages of the following (1) to (3) and a pharmaceutically acceptable excipient:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03513, and (b) lacks an integrase gene and/or an immunity repressor gene, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of the bacteriophage identified by Accession No. NITE BP-03514, and (b) lacks an integrase gene and/or an immunity repressor gene; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of the bacteriophage identified by Accession No. NITE BP-03519.

In the above-described embodiment, the pharmaceutical composition of the present invention comprises bacteriophages of the following (1) to (3):
(1) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03514; and
(3) a bacteriophage having lytic activity against *Mycobacterium avium* and/or *Mycobacterium intracellulare,* wherein a genome of the bacteriophage comprises a nucleic acid sequence of a genome of the bacteriophage identified by Accession No. NITE BP-03519.

In the above-described embodiment, the pharmaceutical composition of the present invention comprises bacteriophages of the following (1) to (3):
(1) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03514; and
(3) a bacteriophage having lytic activity against *Mycobacterium avium* and/or *Mycobacterium intracellulare,* wherein a genome of the bacteriophage consists of a nucleic acid sequence of a genome of the bacteriophage identified by Accession No. NITE BP-03519.

In one embodiment, the pharmaceutical composition of the present invention may include a pharmaceutical composition comprising bacteriophages of the following (1) to (3), and a pharmaceutically acceptable excipient:
(1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and the capsid of the bacteriophage or the passage strain thereof is linked to Inv3 via an S-tag;
(2) a bacteriophage having lytic activity against *Mycobacterium avium* and/or *Mycobacterium intracellulare,* wherein the bacteriophage is a bacteriophage identified by Accession No. NITE BP-03514 or a passage strain thereof; and
(3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.

The pharmaceutical composition of the present invention also may comprise a variant of the bacteriophage identified by any one of Accession Nos. NITE BP-03918, NITE BP-03513, NITE BP-03514, and NITE BP-03519 as long as it has lytic activity against *Mycobacterium avium* and/or *Mycobacterium intracellulare.* Specifically, the pharmaceutical composition of the present invention may comprise variants of the following bacteriophages:
(1) (A) a bacteriophage having lytic activity against *Mycobacterium avium* and/or *Mycobacterium intracellulare,* wherein a genome of the bacteriophage: (a) comprises a nucleic acid sequence that is modified from a nucleic acid sequence of a genome of the bacteriophage identified by Accession No. NITE BP-03918 by deletion, substitution, insertion, or addition, or a combination thereof of 1 to 5000 (e.g., 1 to 1000, 1 to 500, or 1 to 100) nucleotides, and (b) lacks an integrase gene and/or an immunity repressor gene, or (B) the bacteriophage (A) in which the capsid of the bacteriophage is linked further to a cell-penetrating peptide via a tag;
(2) (C) a bacteriophage having lytic activity against *Mycobacterium avium* and/or *Mycobacterium intracellulare,* wherein a genome of the bacteriophage: (a) comprises a nucleic acid sequence that is modified from a nucleic acid sequence of a genome of the bacteriophage identified by Accession No. NITE BP-03513 by deletion, substitution, insertion, or addition, or a combination thereof of 1 to 5000 (e.g., 1 to 1000, 1 to 500, or 1 to 100) nucleotides, and (b) lacks an integrase gene and/or an immunity repressor gene, or (D) the bacteriophage (C) in which the capsid of the bacteriophage is linked further to a cell-penetrating peptide via a tag; and
(3) (E) a bacteriophage having lytic activity against *Mycobacterium avium* and/or *Mycobacterium intracellulare,* wherein a genome of the bacteriophage: (a) comprises a nucleic acid sequence that is modified from a nucleic acid sequence of a genome of the bacteriophage identified by Accession No. NITE BP-03514 by deletion, substitution, insertion, or addition, or a combination thereof of 1 to 5000 (e.g., 1 to 1000, 1 to 500, or 1 to 100) nucleotides, and (b) lacks an integrase gene and/or an immunity repressor gene, or (F) the bacteriophage (E) in which the capsid of the bacteriophage is linked to a cell-penetrating peptide via a tag; and/or
(4) a bacteriophage having lytic activity against *Mycobacterium avium* and/or *Mycobacterium intracellulare,* wherein a genome of the bacteriophage comprises a nucleic acid sequence that is modified from a nucleic acid sequence of a genome of the bacteriophage identified by Accession No. NITE BP-03519 by deletion, substitution, insertion, or addition, or a combination thereof of 1 to 5000 (e.g., 1 to 1000, 1 to 500, or 1 to 100) nucleotides.

For the modifications such as deletion, substitution, insertion, or addition of a nucleotide(s), a plurality of modifications may be continuous, or a plurality of modifications may exist at different positions.

The pharmaceutical composition of the present invention may include a passage strain of a bacteriophage identified by any one of the above-described Accession No. NITE BP-03918, Accession No. NITE BP-03513, Accession No. NITE BP-03514, and Accession No. NITE BP-03519, as long as it has lytic activity against *Mycobacterium avium* and/or *Mycobacterium intracellulare.*

The effective dose, the administration frequency, and the administration period may vary depending on the purpose of administration (therapeutic or preventive purpose), the severity and age of the subject to be administered, the dosage form of the preparation to be used, the potency of the bacteriophage, and the like. For example, about 10⁴ to 10¹⁴ plaque forming unit (pfu) can be used as an effective dose of a single bacteriophage or as a total effective dose of two or more bacteriophages. The dosage ratio of the two or more bacteriophages can be appropriately adjusted according to the severity of symptoms and age of the patient, the dosage form of the preparation to be used, the potency of the bacteriophage, or the like. For example, in the case where the pharmaceutical composition comprises three bacteriophages, each bacteriophage may be contained in approximately equal amounts (e.g., 3.3 × 10³ to 3.3 × 10¹³ pfu), or each bacteriophage may be contained in different ratio.

The pharmaceutical composition of the present invention can be used as an agent for preventing or treating NTM disease, e.g., MAC disease or pulmonary MAC disease.

As used herein, "treat," "treating" and "treatment" mean at least partial improvement of symptoms of NTM disease (e.g., coughing, sputum, bloody sputum, fever, dyspnea, malaise, pulmonary nodules, and bronchiectasis), cessation of progress or deterioration of NTM disease including negative sputum culture, complete cure, and the like. As used herein, "prevent," "preventing" and "prevention" mean preventing a subject not suffering from NTM disease from developing NTM disease, preventing recurrence of NTM disease, and the like.

The present invention may include a pharmaceutical composition for preventing or treating NTM disease, e.g., MAC disease or pulmonary MAC disease, comprising the bacteriophage or the modified bacteriophage of the present invention, or the pharmaceutical composition of the present invention.

Further, the present invention may include a method for preventing or treating NTM disease, e.g., MAC disease or pulmonary MAC disease in a subject, the method comprising a step of administering a therapeutically effective amount of the bacteriophage or the modified bacteriophage of the present invention, or the pharmaceutical composition of the present invention. In one embodiment, at least two, e.g., three bacteriophages or modified bacteriophages of the present invention can be administered to the subject, and the plurality of bacteriophages may be administered simultaneously or separately.

Further, the present invention may include the bacteriophage or the modified bacteriophage of the present invention, or the pharmaceutical composition of the present invention for use in preventing or treating NTM disease, e.g., MAC disease or pulmonary MAC disease. Further, the present invention includes use of the bacteriophage or the modified bacteriophage of the present invention, or the pharmaceutical composition of the present invention in the manufacture of a pharmaceutical composition for preventing or treating NTM disease, e.g., MAC disease or pulmonary MAC disease.

According to the present invention, the subject to which the bacteriophage is administered may not be limited, as long as it is a mammal, and examples thereof may include mice, rats, dogs, pigs, monkeys, and humans. For example, it may be administered to a subject diagnosed with NTM disease or a subject at risk of suffering from NTM disease.

The bacteriophage or the modified bacteriophage of the present invention, or the pharmaceutical composition of the present invention may also be used or administered in combination with other components effective for treating or preventing NTM disease, and the bacteriophage or the modified bacteriophage of the present invention, or the pharmaceutical composition of the present invention may be provided as a combination therapy with such other active components.

The present invention has been generally described above, but specific examples are now provided for reference for further understanding. These examples are for illustrative purposes and are not intended to limit the invention.

### Examples

Unless otherwise specified, the experiment was conducted according to the attached protocol for the parts using commercially available kits or reagents. Moreover, concentration mol/L is represented as M for convenience of description. For example, a 1M sodium hydroxide aqueous solution means a 1 mol/L sodium hydroxide aqueous solution.

### < Example 1: Preparation of new bacteriophage >

A #63 freeze-thaw resistant strain was obtained by the following method with the #63 lysogenic gene-deficient strain (Accession No. NITE BP-03517) used as the parent phage.

In a phage buffer (10 mM Tris-HCl containing 1 mM CaCl₂, 10 mM MgSO₄, 68.4 mM NaCl (pH 7.5)), ethyl methanesulfonate (Nacalai Tesque, Inc.) was added to the #63 lysogenic gene-deficient strain at a final concentration of 33 mM, and the resultant mixture was reacted at 37°C for 1 hour. The solution resulting from the reaction was mixed with *Mycobacterium smegmatis* in soft agar, the resultant was overlaid on a 7H10 plate (19 g/L Middlebrook 7H10 Agar (Difco), 6.3 g/L glycerol, 100 mL/L Middlebrook OADC Enrichment (Difco)), and cultured overnight at 37°C. The phage buffer was added to the plate after the culture, and a supernatant was collected, and then filtered through a 0.22 µm filter. The filtered solution was frozen in a freezer at -80°C, and then thawed at 4°C. This solution was mixed with *Mycobacterium smegmatis* in soft agar, the resultant was cultured by the same procedure as described above, and a filtered supernatant was obtained. The above-described procedure was defined as one cycle, and a total of 12 cycles were performed.

The supernatant obtained after the 12 cycles was cultured by the same procedure as described above, and a #63 freeze-thaw resistant strain was obtained from a single plaque. The Accession No. for this #63 freeze-thaw resistant strain is NITE BP-03918.

### < Example 2: Freeze-thaw resistance activity of #63 freeze-thaw resistant strain >

The freeze-thaw resistance activity of the #63 freeze-thaw resistant strain obtained in Example 1 was confirmed by the following method.

Bacterial colonies of *Mycobacterium smegmatis* grown on a 7H10 plate were scraped off, suspended in LB (Luria-Bertani) medium, and then expanded by culture at 37°C. After the culture, the #63 lysogenic gene-deficient strain or the #63 freeze-thaw resistant strain was added thereto, and the culture was continued at 37°C for about one day.

The supernatant obtained after the culture was filtered through a 0.22 µm filter to yield a phage lysate of each strain. Subsequently, each lysate was purified by ultracentrifugation to obtain a phage solution of the strain.

Purification by ultracentrifugation: DNase I and RNase A were added to the phage lysate of each of the #63 lysogenic gene-deficient strain and the #63 freeze-thaw resistant strain to a final concentration of 1 µg/mL, and the resultant mixture was allowed to stand at room temperature for 30 minutes. NaCl was added to a final concentration of 1 M and dissolved, and the mixture was allowed to stand on ice or in a refrigerator for 1 h. Polyethylene Glycol 8000 was added to a final concentration of 10% w/v and dissolved, and the mixture was allowed to stand on ice or in a refrigerator for 2 h. The supernatant was removed by centrifugation at 11,000 g for 10 min at 4°C, and phage buffer was added to suspend and collect the precipitate. To the collected solution, 0.5 g of cesium chloride per mL was added, gently dissolved and transferred to an ultracentrifuge vessel. Cesium chloride solutions in a concentration of 1.45 g/mL, 1.5 g/mL and 1.7 g/mL each were injected sequentially into the bottom of the vessel using a syringe. The solution was centrifuged under the condition of 22,000 g for 2 h at 4°C, and the band containing the phage was collected. The collected phage-containing solution was replaced with phage buffer by dialysis, thereby preparing a phage solution.

A phage solution of the #63 lysogenic gene-deficient strain or the #63 freeze-thaw resistant strain was diluted and adjusted to a concentration of about 10⁷ pfu/mL. For each phage solution, a sample stored at 4°C and a sample that was frozen at -80°C for 1 hour and then stored at 4°C were prepared.

Bacterial colonies of *Mycobacterium smegmatis* grown on a 7H10 plate were scraped off, and inoculated into a 7H9 medium (4.7 g/L Middlebrook 7H9 Broth (Difco), 100 mL/L Middlebrook OADC Enrichment (Difco), 0.5 g/L polyoxyethylene sorbitan monooleate) and cultured for 3 days. The culture solution was then washed three times with a 7H9 medium excluding polyoxyethylene sorbitan monooleate to prepare a bacterial suspension.

300 µL of the bacterial suspension was mixed with 3 mL of 0.6% soft agar-containing 7H9 medium (4.7 g/L Middlebrook 7H9 Broth (Difco), 6 g/L agarose, 100 mL/L Middlebrook OADC Enrichment (Difco)), and the resultant was overlaid on a 7H10 plate.

Using the phage buffer, a dilution series of six total concentrations was prepared by repeated 10-fold serial dilutions of the group of samples of the #63 lysogenic gene-deficient strain and the #63 freeze-thaw resistant strain prepared as described above. After confirming that the soft agar had completely solidified, 2.5 µL of each dilution was added thereto dropwise, and the resultant was cultured overnight at 37°C.

The results are shown in Figure 1. The pfu of the #63 lysogenic gene-deficient strain (No. 63Δrep, int) decreased by about 1000-fold through the freeze-thaw operation, whereas the pfu of the #63 freeze-thaw resistant strain (No. 63Δrep, int_R12-23) remained substantially the same.

### < Example 3: Production of capsid-modified phage >

A capsid-modified phage was produced by the following method.

In order to express an S-tag fusion capsid structural protein or an S-tag and Inv3 fusion capsid structural protein according to promoter activity, a plasmid was prepared in which a gene encoding an S-tag peptide sequence (SEQ ID NO: 2), or a gene encoding an S-tag peptide sequence and an Inv3 peptide sequence (SEQ ID NO: 1) was inserted by linking upstream or downstream of a gene encoding a capsid structural protein, which was obtained from each of the genomic sequences of the D29 lysogenic gene-deficient strain (Accession No. NITE BP-03513), the B1 lysogenic gene-deficient strain (Accession No. NITE BP-03514), and the #63 freeze-thaw resistant strain obtained in Example 1.

The plasmid was introduced into *Mycobacterium smegmatis* by electroporation to express various fusion capsid structural proteins. During the expression of the fusion capsid protein, the corresponding phage was used for infection as the source of the capsid protein, and thus, a capsid-modified phage having a capsid in which a certain proportion of the fusion capsid structural protein was mixed was produced. The capsid-modified phage was purified using a resin binding to an S-tag (Strep-Tactin XT, IBA GmbH).

By using similar methods, the following various capsid-modified phages were obtained.
- S-tag fusion D29 lysogenic gene-deficient strain (modified phage having a capsid in which an S-tag was fused to the N-terminal side or the C-terminal side of the capsid structural protein. The former is referred to as an S-tag fusion D29 lysogenic gene-deficient strain (N), and the latter is referred to as an S-tag fusion D29 lysogenic gene-deficient strain (C).)
- S-tag and Inv3 fusion D29 lysogenic gene-deficient strain (modified phage having a capsid in which S-tag and Inv3 sequences were fused to the C-terminal side of the capsid structural protein)
- S-tag fusion #63 freeze-thaw resistant strain (modified phage having a capsid in which an S-tag was fused to the C-terminal side of the capsid structural protein)
- S-tag and Inv3 fusion #63 freeze-thaw resistant strain (modified phage having a capsid in which S-tag and Inv3 sequences were fused to the C-terminal side of the capsid structural protein)
- S-tag and Inv3 fusion B1 lysogenic gene-deficient strain (modified phage having a capsid in which S-tag and Inv3 sequences were fused to the C-terminal side of the capsid structural protein)

### < Example 4: Lytic activity of capsid-modified phage >

The lytic activity of each capsid-modified phage was evaluated by the following method.

*Mycobacterium avium* KCH-ASGF-MA-05, or *clarithromycin-resistant Mycobacterium intracellulare* KCH-ASGF-MAC-475 (distributed from Kinki Central Respiratory Centre) grown on a 7H10 plate at 37°C for about 2 weeks was scraped, and suspended in a 7H9 medium (4.7 g/L Middlebrook 7H9 Broth (Difco)). After the suspension, the resultant bacterial suspension was adjusted, with a 7H9 medium, to a final OD600 = 1.5 using a spectrophotometer.

300 µL of the bacterial suspension was mixed with 3 mL of a 7H9 medium containing 0.6% soft agar (4.7 g/L Middlebrook 7H9 Broth (Difco), 6 g/L agarose, and 100 mL/L Middlebrook OADC Enrichment (Difco)), and the resultant mixture was overlaid on a 7H10 plate.

After confirming that the soft agar had completely gelatinized, the S-tag and Inv3 fusion D29 lysogenic gene-deficient strain (D29Δint-Stag-Inv3), the S-tag and Inv3 fusion B1 lysogenic gene-deficient strain (B1Δrep, int-Stag-Inv3), or the S-tag and Inv3 fusion #63 freeze-thaw resistant strain (No. 63Δrep, int _R12-23-Stag-Inv3) obtained in Example 3, or the #63 freeze-thaw resistant strain (No. 63Δrep, int _R12-23) obtained in Example 2 was diluted with a phage buffer to prepare six concentration levels ranging from 10¹⁰ pfu/mL to 10⁵ pfu/mL, and 2.5 µL of the resultant was added dropwise. The resultant was cultured at 37°C for about 9 days. It had been preliminarily confirmed that adding only the phage buffer to the solidified soft agar does not result in plaque formation.

The results are shown in Figure 2. Plaques by all the phages were observed in both the *Mycobacterium avium* KCH-ASGF-MA-05 (A) and the clarithromycin-resistant *Mycobacterium intracellulare* KCH-ASGF-MAC-475 (B). In this manner, it was confirmed that the S-tag and Inv3 fusion D29 lysogenic gene-deficient strain, the S-tag and Inv3 fusion B1 lysogenic gene-deficient strain, and the S-tag and Inv3 fusion #63 freeze-thaw resistant strain, and the #63 freeze-thaw resistant strain obtained in Example 2 had lytic activity against *Mycobacterium avium* and *Mycobacterium intracellulare.*

### < Example 5: Bactericidal activity of capsid-modified phage against intracellular bacterium >

Various phage solutions were obtained by the method of Example 2 or Example 3.

Each of two strains of *Mycobacterium avium* (KCH-ASGF-MA-05 and KCH-ASGF-MA-12 (distributed from Kinki Central Respiratory Centre)) cultured and grown on a 7H10 plate at 37°C for 4 days to about 2 weeks was scraped, and suspended in a cell culture medium. After the suspension, the resultant bacterial suspension was adjusted to a final OD600 = 0.07 using a spectrophotometer. Mouse bronchoalveolar lavage fluid (BALF) was collected, and mouse alveolar macrophages were prepared from the BALF, and then cultured for about 1 week at 37°C in the presence of 5% CO₂. The cultured cells were pooled, and 10⁴ cells per well were seeded onto a 96-well plate. The cells were cultured at 37°C in the presence of 5% CO₂, and after 1 day, the supernatant was replaced with the aforementioned bacterial suspension. After 2 hours, the supernatant of the bacterial suspension was removed, and the resultant cells were washed 3 times with phosphate-buffered saline containing amikacin, and then, a cell culture medium was added thereto, followed by culture for 3 days. After the culture, the supernatant was removed, and each of various phage solutions prepared by the aforementioned method was added thereto to achieve a final concentration of 3.0 × 10⁸ pfu/mL (KCH-ASGF-MA-05) or 1.4 ×10⁹ pfu/mL (KCH-ASGF-MA-12). After culturing for 3 days, the supernatant was removed, and a solution obtained after suspending the resultant by adding a cell lysis buffer thereto was collected for counting CFUs.

The results are shown in Figure 3. All the strains of the bacteriophage had bactericidal activity against intracellular *Mycobacterium avium* KCH-ASGF-MA-05, and the S-tag fusion D29 lysogenic gene-deficient strain (N) and the S-tag fusion D29 lysogenic gene-deficient strain (C) exhibited higher bactericidal activity than the D29 lysogenic gene-deficient strain (Figure 3A). Both the strains of the bacteriophage had bactericidal activity against intracellular *Mycobacterium avium* KCH-ASGF-MA-12, and the S-tag fusion #63 freeze-thaw resistant strain exhibited higher bactericidal activity than the #63 freeze-thaw resistant strain (Figure 3B). In this manner, it was confirmed that the S-tag fusion D29 lysogenic gene-deficient strain (N), the S-tag fusion D29 lysogenic gene-deficient strain (C), and the S-tag fusion #63 freeze-thaw resistant strain have high lytic activity *against Mycobacterium avium.*

### < Example 6: Bactericidal activity of respective bacteriophage cocktails against intracellular bacterium >

The B1 lysogenic gene-deficient strain (Accession No. NITE BP-03514), and the #123 parent strain (Accession No. NITE BP-03519) were purified by ultracentrifugation. The method of Example 5 was employed for evaluating the bactericidal activity of various bacteriophage cocktails against intracellular *Mycobacterium avium* KCH-ASGF-MA-05.

The bacteriophage cocktails used were the following three types, each of which was a cocktail prepared by mixing equal volumes of three phages at a final concentration of 1.0 × 10⁹ pfu/mL.
- Cocktail #1:
   S-tag and Inv3 fusion D29 lysogenic gene-deficient strain, B1 lysogenic gene-deficient strain, and #123 parent strain
- Cocktail #2:
   S-tag and Inv3 fusion D29 lysogenic gene-deficient strain, S-tag and Inv3 fusion #63 freeze-thaw resistant strain, and B1 lysogenic gene-deficient strain
- Cocktail #3:
   S-tag and Inv3 fusion D29 lysogenic gene-deficient strain, S-tag and Inv3 fusion #63 freeze-thaw resistant strain, and #123 parent strain

The results are shown in Figure 4. It was revealed that all the bacteriophage cocktails tested have bactericidal activity against intracellular *Mycobacterium avium* KCH-ASGF-MA-05.

### < Example 7: Bactericidal activity of capsid-modified phage against intracellular bacterium >

The D29 lysogenic gene-deficient strain (Accession No. NITE BP-03513) and the B1 lysogenic gene-deficient strain (Accession No. NITE BP-03514) were purified by ultracentrifugation to obtain phage solutions thereof. The S-tag and Inv3 fusion D29 lysogenic gene-deficient strain and the S-tag and Inv3 fusion B1 lysogenic gene-deficient strain were purified by the method of Example 3 to obtain phage solutions thereof.

The bactericidal activity of the various bacteriophages against intracellular *Mycobacterium avium* KCH-ASGF-MA-05 was evaluated by the method of Example 5. The phage solution was added to achieve a final concentration of the bacteriophage of 3.0 × 10⁹ pfu/mL.

The results are shown in Figure 5. All the strains of the bacteriophage had bactericidal activity against intracellular *Mycobacterium avium* KCH-ASGF-MA-05. The S-tag and Inv3 fusion D29 lysogenic gene-deficient strain exhibited higher bactericidal activity than the D29 lysogenic gene-deficient strain (Figure 5A), and the S-tag and Inv3 fusion B1 lysogenic gene-deficient strain exhibited higher bactericidal activity than the B1 lysogenic gene-deficient strain (Figure 5B).

All publications, patents, and patent applications cited herein are incorporated by reference in their entirety.

### Industrial Applicability

The bacteriophage of the present invention and the modified bacteriophage of the present invention, and the pharmaceutical composition are expected to be useful for preventing or treating nontuberculous mycobacterial disease, such as MAC disease or pulmonary MAC disease.

### [Accession Nos.]

Accession No. NITE BP-03513 (bacteriophage D29 lysogenic gene-deficient strain D29Δ, deposited on August 26, 2021)
Accession No. NITE BP-03514 (bacteriophage B1 lysogenic gene-deficient strain B1Δ, deposited on August 26, 2021)
Accession No. NITE BP-03519 (bacteriophage #123 parent strain, deposited on August 26, 2021)
Accession No. NITE BP-03918 (bacteriophage #63 freeze-thaw resistant strain, deposited on June 22, 2023)

### Sequence Listing Free Text

SEQ ID NO: 1: synthetic construct (Inv3)
SEQ ID NO: 2: synthetic construct (S-tag)

## Claims

1. A bacteriophage having lytic activity against *Mycobacterium avium* and/or *Mycobacterium intracellulare,*
wherein a genome of the bacteriophage: (a) comprises a nucleic acid sequence having 90% or more identity to a nucleic acid sequence of a genome of a bacteriophage identified by Accession No. NITE BP-03513, and (b) lacks an integrase gene and/or an immunity repressor gene, and
a capsid of the bacteriophage is linked to an S-tag.

2. The bacteriophage according to claim 1, wherein the genome of the bacteriophage comprises the nucleic acid sequence of the genome of the bacteriophage identified by Accession No. NITE BP-03513.

3. The bacteriophage according to claim 1 or 2, wherein the genome of the bacteriophage consists of the nucleic acid sequence of the genome of the bacteriophage identified by Accession No. NITE BP-03513.

4. A bacteriophage having lytic activity against *Mycobacterium avium* and/or *Mycobacterium intracellulare,* wherein the bacteriophage is a bacteriophage identified by Accession No. NITE BP-03513 or a passage strain thereof, and a capsid of the bacteriophage or the passage strain thereof is linked to an S-tag.

5. The bacteriophage according to any one of claims 1 to 4, wherein the capsid is linked further to a cell-penetrating peptide via the S-tag.

6. The bacteriophage according to claim 5, wherein the cell-penetrating peptide comprises Inv3.

7. A pharmaceutical composition comprising the bacteriophage according to any one of claims 1 to 6, and a pharmaceutically acceptable excipient.

8. The pharmaceutical composition according to claim 7, further comprising one or more strains of a bacteriophage having lytic activity against *Mycobacterium avium* and/or *Mycobacterium intracellulare.*

9. A bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03918, and (b) lacks an integrase gene and/or an immunity repressor gene.

10. The bacteriophage according to claim 9, which possesses resistance to freeze-thaw.

11. The bacteriophage according to claim 9, wherein the genome of the bacteriophage comprises the nucleic acid sequence of the genome of the bacteriophage identified by Accession No. NITE BP-03918.

12. The bacteriophage according to claim 9 or 11, wherein the genome of the bacteriophage consists of the nucleic acid sequence of the genome of the bacteriophage identified by Accession No. NITE BP-03918.

13. A bacteriophage having lytic activity against *Mycobacterium avium* and/or *Mycobacterium intracellulare,* wherein the bacteriophage is a bacteriophage identified by Accession No. NITE BP-03918 or a passage strain thereof.

14. The bacteriophage according to any one of claims 9 to 13, wherein a capsid of the bacteriophage is linked to a tag.

15. The bacteriophage according to any one of claims 9 to 13, wherein a capsid of the bacteriophage is linked to a cell-penetrating peptide via a tag.

16. The bacteriophage according to claim 14 or 15, wherein the tag is an S-tag.

17. The bacteriophage according to claim 15 or 16, wherein the cell-penetrating peptide comprises Inv3.

18. A pharmaceutical composition comprising the bacteriophage according to any one of claims 9 to 17, and a pharmaceutically acceptable excipient.

19. The pharmaceutical composition according to claim 18, further comprising one or more strains of a bacteriophage having lytic activity against *Mycobacterium avium* and/or *Mycobacterium intracellulare.*

20. The pharmaceutical composition according to claim 7, comprising the bacteriophage according to any one of claims 1 to 6, and further comprising one or more bacteriophages selected from the group consisting of bacteriophages of the following (1) to (3):
(1) (A) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03514, and (b) lacks an integrase gene and/or an immunity repressor gene, or (B) the bacteriophage (A) in which a capsid of the bacteriophage is linked to a cell-penetrating peptide via a tag;
(2) (C) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03918, and (b) lacks an integrase gene and/or an immunity repressor gene, or (D) the bacteriophage (C) in which a capsid of the bacteriophage is linked to a cell-penetrating peptide via a tag; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of a bacteriophage identified by Accession No. NITE BP-03519.

21. The pharmaceutical composition according to claim 7, comprising the bacteriophage according to any one of claims 1 to 6, and further comprising one or more bacteriophages selected from the group consisting of bacteriophages of the following (1) to (3):
(1) (A) a bacteriophage having lytic activity against *Mycobacterium avium* and/or *Mycobacterium intracellulare,* wherein a genome of the bacteriophage comprises a nucleic acid sequence of a genome of a bacteriophage identified by Accession No. NITE BP-03514, or (B) the bacteriophage (A) in which a capsid of the bacteriophage is linked to a cell-penetrating peptide via a tag;
(2) (C) a bacteriophage having lytic activity against *Mycobacterium avium* and/or *Mycobacterium intracellulare,* wherein a genome of the bacteriophage comprises a nucleic acid sequence of a genome of a bacteriophage identified by Accession No. NITE BP-03918, or (D) the bacteriophage (C) in which a capsid of the bacteriophage is linked to a cell-penetrating peptide via a tag; and
(3) a bacteriophage having lytic activity against *Mycobacterium avium* and/or *Mycobacterium intracellulare,* wherein a genome of the bacteriophage comprises a nucleic acid sequence of a genome of a bacteriophage identified by Accession No. NITE BP-03519.

22. The pharmaceutical composition according to claim 7, comprising the bacteriophage according to any one of claims 1 to 6, and further comprising one or more bacteriophages selected from the group consisting of bacteriophages of the following (1) to (3):
(1) (A) a bacteriophage having lytic activity against *Mycobacterium avium* and/or *Mycobacterium intracellulare,* wherein a genome of the bacteriophage consists of a nucleic acid sequence of a genome of a bacteriophage identified by Accession No. NITE BP-03514, or (B) the bacteriophage (A) in which a capsid of the bacteriophage is linked to a cell-penetrating peptide via a tag;
(2) (C) a bacteriophage having lytic activity against *Mycobacterium avium* and/or *Mycobacterium intracellulare,* wherein a genome of the bacteriophage consists of a nucleic acid sequence of a genome of a bacteriophage identified by Accession No. NITE BP-03918, or (D) the bacteriophage (C) in which a capsid of the bacteriophage is linked to a cell-penetrating peptide via a tag; and
(3) a bacteriophage having lytic activity against *Mycobacterium avium* and/or *Mycobacterium intracellulare,* wherein a genome of the bacteriophage consists of a nucleic acid sequence of a genome of a bacteriophage identified by Accession No. NITE BP-03519.

23. The pharmaceutical composition according to claim 7, comprising the bacteriophage according to any one of claims 1 to 6, and further comprising one or more bacteriophages selected from the group consisting of bacteriophages of the following (1) to (3):
(1) (A) a bacteriophage having lytic activity against *Mycobacterium avium* and/or *Mycobacterium intracellulare,* wherein the bacteriophage is a bacteriophage identified by Accession No. NITE BP-03514 or a passage strain thereof, or (B) the bacteriophage (A) in which a capsid of the bacteriophage or the passage strain thereof is linked to a cell-penetrating peptide via a tag;
(2) (C) a bacteriophage having lytic activity against *Mycobacterium avium* and/or *Mycobacterium intracellulare,* wherein the bacteriophage is a bacteriophage identified by Accession No. NITE BP-03918 or a passage strain thereof, or (D) the bacteriophage (C) in which a capsid of the bacteriophage or the passage strain thereof is linked to a cell-penetrating peptide via a tag; and
(3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.

24. A pharmaceutical composition comprising bacteriophages of the following (1) to (3), and a pharmaceutically acceptable excipient:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03513, and (b) lacks an integrase gene and/or an immunity repressor gene, and a capsid of the bacteriophage is linked to Inv3 via an S-tag;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03514, and (b) lacks an integrase gene and/or an immunity repressor gene; and
(3) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03918, and (b) lacks an integrase gene and/or an immunity repressor gene, and a capsid of the bacteriophage is linked to Inv3 via an S-tag.

25. The pharmaceutical composition according to claim 24, comprising bacteriophages of the following (1) to (3):
(1) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03514; and
(3) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03918, and the capsid of the bacteriophage is linked to Inv3 via an S-tag.

26. The pharmaceutical composition according to claim 24 or 25, comprising bacteriophages of the following (1) to (3):
(1) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03514; and
(3) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03918, and the capsid of the bacteriophage is linked to Inv3 via an S-tag.

27. A pharmaceutical composition comprising bacteriophages of the following (1) to (3), and a pharmaceutically acceptable excipient:
(1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and a capsid of the bacteriophage or the passage strain thereof is linked to Inv3 via an S-tag;
(2) a bacteriophage having lytic activity against *Mycobacterium avium* and/or *Mycobacterium intracellulare,* wherein the bacteriophage is a bacteriophage identified by Accession No. NITE BP-03514 or a passage strain thereof; and
(3) a bacteriophage having lytic activity against *Mycobacterium avium* and/or *Mycobacterium intracellulare,* wherein the bacteriophage is a bacteriophage identified by Accession No. NITE BP-03918 or a passage strain thereof, and a capsid of the bacteriophage or the passage strain thereof is linked to Inv3 via an S-tag.

28. A pharmaceutical composition comprising bacteriophages of the following (1) to (3), and a pharmaceutically acceptable excipient:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03513, and (b) lacks an integrase gene and/or an immunity repressor gene, and a capsid of the bacteriophage is linked to Inv3 via an S-tag;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03918, and (b) lacks an integrase gene and/or an immunity repressor gene, and a capsid of the bacteriophage is linked to Inv3 via an S-tag; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of a bacteriophage identified by Accession No. NITE BP-03519.

29. The pharmaceutical composition according to claim 28, comprising bacteriophages of the following (1) to (3):
(1) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03918, and the capsid of the bacteriophage is linked to Inv3 via an S-tag; and
(3) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03519.

30. The pharmaceutical composition according to claim 28 or 29, comprising bacteriophages of the following (1) to (3):
(1) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03918, and the capsid of the bacteriophage is linked to Inv3 via an S-tag; and
(3) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03519.

31. A pharmaceutical composition comprising bacteriophages of the following (1) to (3), and a pharmaceutically acceptable excipient:
(1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and a capsid of the bacteriophage or the passage strain thereof is linked to Inv3 via an S-tag;
(2) a bacteriophage having lytic activity against *Mycobacterium avium* and/or *Mycobacterium intracellulare,* wherein the bacteriophage is a bacteriophage identified by Accession No. NITE BP-03918 or a passage strain thereof, and a capsid of the bacteriophage or the passage strain thereof is linked to Inv3 via an S-tag; and
(3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.

32. A pharmaceutical composition comprising bacteriophages of the following (1) to (3), and a pharmaceutically acceptable excipient:
(1) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03513, and (b) lacks an integrase gene and/or an immunity repressor gene, and a capsid of the bacteriophage is linked to Inv3 via an S-tag;
(2) a bacteriophage having lytic activity against *Mycobacterium avium* and/or *Mycobacterium intracellulare,* wherein a genome of the bacteriophage: (a) comprises a nucleic acid sequence having 90% or more identity to a nucleic acid sequence of a genome of a bacteriophage identified by Accession No. NITE BP-03514, and (b) lacks an integrase gene and/or an immunity repressor gene; and
(3) a bacteriophage having lytic activity against *Mycobacterium avium* and/or *Mycobacterium intracellulare,* wherein a genome of the bacteriophage comprises a nucleic acid sequence having 90% or more identity to a nucleic acid sequence of a genome of a bacteriophage identified by Accession No. NITE BP-03519.

33. The pharmaceutical composition according to claim 32, comprising bacteriophages of the following (1) to (3):
(1) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03514; and
(3) a bacteriophage having lytic activity against *Mycobacterium avium* and/or *Mycobacterium intracellulare,* wherein a genome of the bacteriophage comprises the nucleic acid sequence of the genome of the bacteriophage identified by Accession No. NITE BP-03519.

34. The pharmaceutical composition according to claim 32 or 33, comprising bacteriophages of the following (1) to (3):
(1) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03513, and the capsid of the bacteriophage is linked to Inv3 via an S-tag;
(2) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03514; and
(3) a bacteriophage having lytic activity against *Mycobacterium avium* and/or *Mycobacterium intracellulare,* wherein a genome of the bacteriophage consists of the nucleic acid sequence of the genome of the bacteriophage identified by Accession No. NITE BP-03519.

35. A pharmaceutical composition comprising bacteriophages of the following (1) to (3), and a pharmaceutically acceptable excipient:
(1) a bacteriophage having lytic activity against *Mycobacterium avium* and/or *Mycobacterium intracellulare,* wherein the bacteriophage is a bacteriophage identified by Accession No. NITE BP-03513 or a passage strain thereof, and a capsid of the bacteriophage or the passage strain thereof is linked to Inv3 via an S-tag;
(2) a bacteriophage having lytic activity against *Mycobacterium avium* and/or *Mycobacterium intracellulare,* wherein the bacteriophage is a bacteriophage identified by Accession No. NITE BP-03514 or a passage strain thereof; and
(3) a bacteriophage having lytic activity against *Mycobacterium avium* and/or *Mycobacterium intracellulare,* wherein the bacteriophage is a bacteriophage identified by Accession No. NITE BP-03519 or a passage strain thereof.

36. The pharmaceutical composition according to any one of claims 7, 8, 18, 19, and 20 to 35, which is a pharmaceutical composition for preventing or treating nontuberculous mycobacterial disease.

37. The pharmaceutical composition according to claim 36, wherein the nontuberculous mycobacterial disease is pulmonary MAC disease.
